(19) **Europäisches Patentamt European Patent Office Office européen des brevets**

(11) **EP 3 588 382 B1**

(12) **EUROPEAN PATENT SPECIFICATION**

(45) Date of publication and mention of the grant of the patent:
**25.02.2026 Bulletin 2026/09**

(51) International Patent Classification (IPC):
**G06V 10/82** (2022.01)    **G06V 10/764** (2022.01)

(52) Cooperative Patent Classification (CPC):
**G06V 10/764; G06V 10/82; G06V 2201/03**

(21) Application number: **19182560.3**

(22) Date of filing: **26.06.2019**

(54) **A DEEP LEARNING METHOD FOR TUMOR CELL SCORING ON CANCER BIOPSIES**

TIEFLERNVERFAHREN ZUR TUMORZELLBEWERTUNG AN KREBSBIOPSIEN

PROCÉDÉ D'APPRENTISSAGE PROFOND POUR LA NOTATION DE CELLULES TUMORALES LORS DES BIOPSIES DU CANCER

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **26.06.2018 US 201862690329 P**

(43) Date of publication of application:
**01.01.2020 Bulletin 2020/01**

(73) Proprietor: **AstraZeneca Computational Pathology GmbH 80636 München (DE)**

(72) Inventors:
• **KAPIL, Ansh 80339 München (DE)**
• **BRIEU, Nicolas 80649 München (DE)**

(74) Representative: **AstraZeneca Intellectual Property Eastbrook House Shaftesbury Road Cambridge CB2 8BF (GB)**

(56) References cited:
• RIKU TURKKI ET AL: "Antibody-supervised deep learning for quantification of tumor-infiltrating immune cells in hematoxylin and eosin stained breast cancer samples", JOURNAL OF PATHOLOGY INFORMATICS, 1 September 2016 (2016-09-01), XP055587403, Retrieved from the Internet <URL:https://www.ncbi.nlm.nih.gov/pmc/articles/PMC5027738/> [retrieved on 20190509], DOI: 10.4103/2153-3539.189703
• LE HOU ET AL: "Patch-Based Convolutional Neural Network for Whole Slide Tissue Image Classification", 9 March 2016 (2016-03-09), XP055523985, Retrieved from the Internet <URL:https://arxiv.org/pdf/1504.07947.pdf> [retrieved on 20181114]
• LI ZEJU ET AL: "Brain Tumor Segmentation Using an Adversarial Network", 17 February 2018, INTELLIGENT VIRTUAL AGENT. IVA 2015. LNCS; [LECTURE NOTES IN COMPUTER SCIENCE; LECT.NOTES COMPUTER], SPRINGER, BERLIN, HEIDELBERG, PAGE(S) 123 - 132, ISBN: 978-3-642-17318-9, XP047464663
• HAVAEI MOHAMMAD ET AL: "Brain tumor segmentation with Deep Neural Networks", MEDICAL IMAGE ANALYSIS, vol. 35, 1 January 2017 (2017-01-01), pages 18 - 31, XP029801693, ISSN: 1361-8415, DOI: 10.1016/J.MEDIA.2016.05.004
• CARLOS X HERN\'ANDEZ ET AL: "Using Deep Learning for Segmentation and Counting within Microscopy Data", ARXIV.ORG, CORNELL UNIVERSITY LIBRARY, 201 OLIN LIBRARY CORNELL UNIVERSITY ITHACA, NY 14853, 28 February 2018 (2018-02-28), XP081213794

EP 3 588 382 B1

**Description**

TECHNICAL FIELD

The present invention relates to a method for generating a score of a histological diagnosis of a cancer patient by training a model to determine how many pixels of a digital image belong to a tissue that has been positively stained by a diagnostic antibody.

BACKGROUND

Identifying tumor regions in digital images of cancer tissue is often a prerequisite to performing diagnostic and treatment measures, such as classifying cancers using standard grading schemes. The digital images of tissue slices used in histopathology are very large. Individual images require gigabytes to store. Manual annotation of the tumor regions in whole slides through the visual assessment of a pathologist is laborious considering the high volume of data. Therefore, "chemical annotation" has been used to substitute the marking of tumor regions by a pathologist with image recognition of regions stained by biomarkers that identify tissue that tends to be cancerous. Annotating tumor regions using specific antibody staining decreases the subjectivity of the pathologist's evaluation and accelerates the otherwise tedious process. Immunohistochemical (IHC) staining can be used to distinguish marker-positive cells that express a particular protein from marker-negative cells that do not express the protein. IHC staining typically involves multiple dyes, which includes one or more dyes connected to proteinspecific antibodies and another dye that is a counterstain. A common counterstain is hematoxylin, which labels DNA and thus stains nuclei.

A protein specific stain or biomarker can be used to identify the regions of the tissue that are likely cancerous. For example, a biomarker that stains epithelial cells can help to identify the suspected tumor regions. Then other protein specific biomarkers are used to characterize the cancerous tissue. The regions stained by a specific biomarker can be identified and quantified and subsequently a score indicating the amount of positively stained tissue and negatively stained tissue can be visually estimated by pathologists. However, visual assessment by pathologists is prone to variability and subjectivity.

Thus, a computer-based method is sought for generating a repeatable and objective score of a histological diagnosis of a cancer patient, based on a precise estimation of tissue stained by a diagnostic antibody labeling cells that express a specific, cancertreatment-related protein.

SUMMARY

The disclosed method uses a convolutional neural network model to determine how many pixels of an image patch that is cropped from a digital image of an immunohistochemically stained tissue belong to tissue that has been positively stained by a diagnostic antibody. The tissue that has been positively stained by the diagnostic antibody can be a specific cell, a specific group of cells or a specific type of cells present in the immunohistochemically stained tissue, for example, a macrophage or an epithelial cell or another cell that positively stains for the diagnostic antibody.

In a first step, a first image patch that is cropped from a digital image of a tissue slice immunohistochemically stained using a diagnostic antibody is loaded into a processing unit. In a second step, the first image patch is processed using a lconvolutiona neural network to determine how many pixels of the first image patch belong to a first tissue that both belongs to tumor epithelium and that has been positively stained using the diagnostic antibody. The pixel-wise analysis of the image patch allows for high spatial resolution and precision in identifying tumor epithelium tissue that is positively stained using the diagnostic antibody. In a third step, additional image patches that have been cropped from the digital image are processed to determine how many pixels of each additional image patch belong to the first tissue. Then, the score of the histopathological diagnosis is computed based on the total number of pixels of the digital image that belong to the first tissue. The digital image and the score are displayed on a graphical user interface. In some embodiments, the score is the Tumor Cell (TC) score.

Furthermore, the method includes performing image processing on an image patch using a generative adversarial network (GAN) to train a convolutional neural network to determine how many pixels of the image patch belong to (a) a first tissue that is both tumor epithelium and has been positively stained using a diagnostic antibody, (b) a second tissue that is both tumor epithelium and has been negatively stained using the diagnostic antibody, or (c) a third tissue that is neither the first tissue nor the second tissue. Consequently, both the first tissue and the second tissue are tumor epithelium. In one aspect, the second tissue is considered to be negatively stained if the tissue is not positively stained using the diagnostic antibody. Then, the score of the histopathological diagnosis is computed based on the total number of pixels that belong to the first tissue. The digital image and the score are displayed on a graphical user interface.

The convolutional neural network is trained using a generative adversarial network that transforms image patches generated on a stain domain A into fake patches of a stain domain B. A stain domain refers to the region of a digital image of tissue that has been stained for a specific biomarker. For example, the stain domain A is the tissue or the region of a digital image of a tissue that stains for a specific biomarker or stain such as cytokeratin (CK). The stain domain B, for example, is the tissue or the region of the digital image of the tissue that stains for a different specific biomarker or stain, such as programmed death ligand 1 (PD-L1). The generative adversarial network then transforms image patches generated by CK staining into fake patches that are realistic fakes of PD-L1 staining.

Furthermore, a generative adversarial network is used to train a convolutional neural network to transform image patches generated on a stain domain A into fake patches of a stain domain B and then to perform segmentation on the stain domain B. For example, the generative adversarial network performs segmentation on the fake PD-L1 patches generated using CK staining to make realistic fakes of PD-L1 staining.

Furthermore, the method involves training a convolutional neural network using two generative adversarial networks. A first of the two generative adversarial networks transforms image patches generated on a stain domain A into fake patches of a stain domain B. A second of the two generative adversarial networks transforms image patches generated on the stain domain B into fake patches of the stain domain A. For example, the first generative adversarial network transforms image patches generated by CK staining into fake patches that are realistic fakes of PD-L1 staining, and the second generative adversarial network transforms image patches generated using PD-L1 staining into fake patches that are realistic fakes of CK staining.

Another aspect of the disclosure concerns a system that generates a histopathological diagnosis for a cancer patient. The diagnostic system includes code that loads an image patch into a processing unit. The image patch is cropped from a digital image of a tissue slice. The image was acquired by scanning cancer tissue that was immunohistochemically stained using a diagnostic antibody. The system also includes code that processes the image patch using a convolutional neural network to determine whether each pixel of the image patch belongs to (a) a tumor epithelium tissue that has been positively stained using the diagnostic antibody, (b) a tumor epithelium tissue that was negatively stained using the diagnostic antibody, or (c) other tissue. The system also includes code for processing multiple image patches cropped from the image so as to compute a score for the histopathological diagnosis based on a total number of pixels determined to belong to the tumor epithelium tissue that was positively stained using the diagnostic antibody. The image and the score are then displayed on a graphical user interface of the system.

Other embodiments and advantages are described in the detailed description below. This summary does not purport to define the invention. The invention is defined by the claims.

## BRIEF DESCRIPTION OF THE DRAWINGS

The accompanying drawings, where like numerals indicate like components, illustrate embodiments of the invention.

FIG. 1 is a diagram of a novel system for generating a histopathological diagnosis for a cancer patient by computing a sore for the histopathological diagnosis based on a total number of pixels determined to belong to tumor epithelium tissue that has been positively stained by a diagnostic antibody.

FIG. 2 illustrates the process of acquiring a digital image of a slice of tissue from a cancer patient that is stained with a diagnostic antibody, and the process of acquiring a digital image of a slice of tissue from another cancer patient that is stained with an antibody specific for epithelium.

FIG. 3 is a digital image showing epithelial cells have been stained with cytokeratin (CK) and nuclei that have been stained with hematoxylin.

FIG. 4 shows the image of FIG. 3 in which the regions stained positively by CK are identified and segmented.

FIG. 5 is a digital image showing tissue that has been stained with a diagnostic antibody that stains PD-L1 and thus identifies regions in which the cells are positively stained epithelium, not-positively stained epithelium and non-epithelial.

FIG. 6 shows the image of FIG. 5 with the region that is positively stained tumor epithelium marked with cross hatching, the region that is not not-positively stained tumor epithelium marked with horizontal lines and the region that is non-tumor epithelial tissue marked with vertical lines.

FIG. 7 illustrates (i) a convolutional neural network used to determine how many pixels of a first image patch belong to a first tissue that both belongs to tumor epithelium and that has been positively stained using a diagnostic antibody, and (ii) a generative adversarial network used to train the convolutional neural network.

FIG. 8 shows in more detail the network architecture of the generative adversarial network (GAN) used to train the convolutional neural network.

FIG. 9A is a real image of epithelial tissue with regions that have been positively stained with CK.

FIG. 9B is a synthetic image representing epithelial tissue positively stained with PD-L1 that was generated from the image of FIG. 9A by a convolutional neural network trained by a generative adversarial network.

FIG. 9C is a synthetic image representing epithelial tissue not positively stained with PD-L1 that was generated from the image of FIG. 9A.

FIG. 10A is a second real image of epithelial tissue with regions that have been positively stained with CK.

FIG. 10B is a second fake image representing epithelial tissue positively stained with PD-L1 that was generated from the real image of FIG. 10A.

FIG. 10C is another fake image representing epithelial tissue not positively stained with PD-L1 that was generated from the real image of FIG. 10A.

FIG. 10D is an overview of the synthetic (a) and real (b) PD-L1 datasets generated from the analysis of CK stained images and manual annotations respectively. FIG. 10D (c) is an overview of the proposed DASGAN model for joint domain adaptation and semantic segmentation. Pixelwise mask conditioning is added to the generators $G_{AB}$ and $G_{BA}$. The discriminators $D_A$ and $D_B$ are designed with a U-Net like architecture in order to predict both the source of the image (real/synthetic) and to perform the segmentation task.

FIG. 11 shows segmentation accuracy on the validation set, for the three baseline models (in gray, blue and yellow) and for the proposed DASGAN model (in orange) under the condition (i) of low availability of manual annotations on real PD-L1 images. F1 scores are reported for each class of interest - tumor epithelium (TC), tumor epithelium positive (TC+), tumor epithelium negative (TC-) and other, together with their average score Avg. in both scenarios of tumor epithelium detection and replication of TC score.

FIG. 12 shows segmentation accuracy on the validation set (avg. f1-score), for the baseline model trained only on real PD-L1 samples and for the proposed DASGAN model trained on both real and synthetic PD-L1 samples, for increasing availability (i)-(ii)-(iii) of manual annotations on real PD-L1 images.

FIG. 13 shows (a) an example of negative (red) and positive (blue) tumor epithelial regions as well as of non-epithelial regions (green) segmented by the proposed DASGAN model. FIG. 13(b) shows a bar plot showing mean and standard deviation of the TC scores estimated by the proposed approach on unseen cases (n=704), on the following TC score bins: $TC < 1$, $1 <= TC < 10$, $10n <= TC < 10(n + 1)$ for $1 < n < 10$ and $TC = 100$. The inverted histogram at the top shows the distribution of cases w.r.t. pathologist TC score.

FIG. 14 shows the proposed AC-GAN architecture. The noise z and the class one-hot encoding c are concatenated and sent to the generator (G). The generator creates classconditioned fake images. The classes c and the source information s are jointly predicted by the discriminator (D) given a fake (left) or a real (right) input. $SNC_{onv}$ implies convolutional layers using spectral normalization, which introduces a minor modification to the original AC-GAN architecture.

FIG. 15 shows an example of class probability maps obtained with AC-GAN model. Left: Original images. Right: Predicted probabilities associated to positive tumor cell region TC(+) (red channel), negative tumor cell region (green channel) and to other classes (blue channel) superimposed to the original grayscale layer. The two yellow boxes overlaid to the far left images correspond to the regions of interest displayed on the right.

FIG. 16 shows concordance measures between the consolidated visual TC score and the TC scores automatically estimated from the tumor regions detected using the different supervised and semi-supervised network architectures: fully-supervised shallow VGG net (FS-VGG), fully-supervised inception net v2 (FS-InceptionV2), semi-supervised VGG net (SSL-VGG), semi-supervised inception net v2 (SSL-InceptionV2), and semi-supervised AC-GAN (SLL-ACGAN). Values are computed on the $N_{test}$ cases unseen during training or testing of the networks.

FIG. 17 shows a pairwise scatter plot between three visual TC scores where each disk represents one of the 60 visually scored slides. For a given case, the size of the disk is proportional to the inter-rater variability $\Delta_{path}$.

FIG. 18 shows concordance measures between the consolidated visual TC score and the TC scores automatically estimated from the tumor regions detected using the different supervised and semi-supervised network architectures, for increasing maximum levels of inter-rater variability $\Delta_{patho}$ on the x-axis. The green curve indicates the percentage of slides for which the inter-rater variability stays below the given level.

FIG. 19 shows, considering the three visual TC scores and the TC score estimated using the region detected by the AC-GAN network, concordance measures between each TC score and the median of the three remaining TC scores for increasing maximum levels of inter-rater variability $\Delta_{path}$. on the x-axis. The green curve indicates the percentage of slides for which the inter-rater variability stays below the given level.

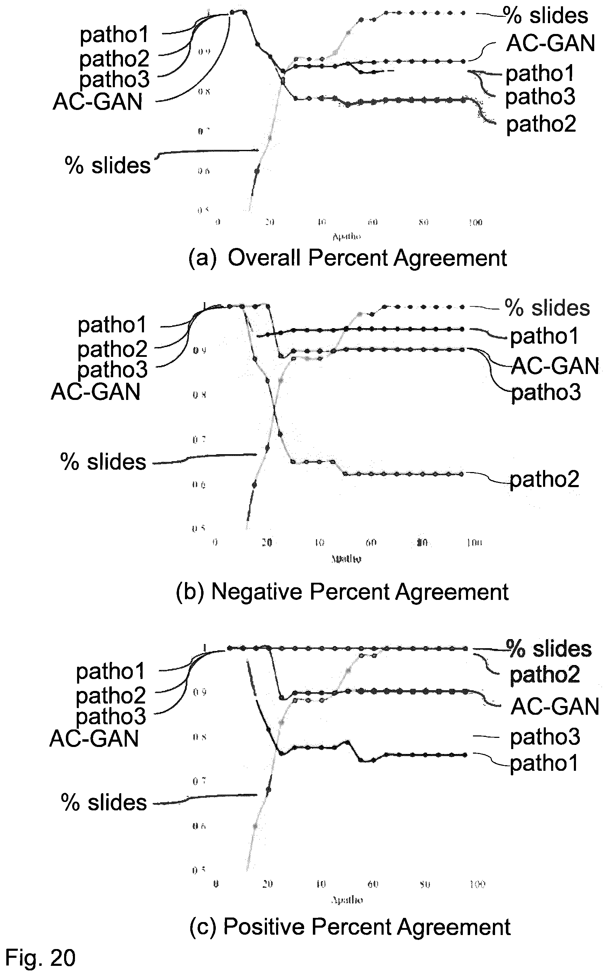

FIG. 20 shows, considering the three visual TC scores and the TC score estimated using the region detected by the AC-GAN network, concordance measures between the patient status estimated from each TC score and patient status estimated from the median of the three remaining TC scores for increasing maximum levels of inter-rater variability $\Delta_{path}$. on the x-axis. The green curve indicates the percentage of slides for which the inter-rater variability stays below the given level.

FIG. 21 shows an overview of the method of generating a Tumor Cell (TC) score of a cancer patient.

FIG. 22 shows the variability of region annotation of tumor tissue biopsy slides between two pathologists.

FIG. 23 relates to Strongly Supervised Learning (TC, tumor cell).

FIG. 24 relates to Semi-Supervised Learning.

FIG. 25 relates to classification networks.

FIG. 26 relates to autoencoders.

FIG. 27 relates to semi-supervised autoencoders.

FIG. 28 relates to Generative Adversarial Networks (GANs).

FIG. 29 shows generated artificial but real-looking PD-L1 images.

FIG. 30 relates to Auxiliary Classifier GANs (AC-GANs) .

FIG. 31 relates to Semantic Segmentation Networks.

FIG. 32 relates to Regression Networks.

FIG. 33 shows Generator loss, Discriminator loss and Classifier loss.

FIG. 34 shows an example of region segmentation.

FIG. 35 shows another example of region segmentation

FIG. 36 relates to inter-pathologist variability.

FIG. 37 shows the results of computing Lin and Pearson correlations for increasing value of allowed inter-pathologist variabilty ($\Delta$patho).

FIG. 38 shows the Lin and Pearson correlations for $\Delta$patho < 30.

FIG. 39 shows the results of comparing the semi-supervised model AC-GAN with a pathologist.

FIG. 40 shows the results of computing Lin concordance (LinCC) and Pearson correlation (PearsonCC) for the three human pathologists and the AC-GAN model.

FIG. 41 shows the Lin concordances and Pearson correlations.

FIG. 42 shows the results of computing Overall Percent Agreement (OPA), Negative Percent Agreement (NPA), and Positive Percent Agreement (PPA) for the three human pathologists and the AC-GAN model.

FIG. 43 shows the Overall Percent Agreement (OPA), Negative Percent Agreement (NPA), and Positive Percent Agreement (PPA).

## DETAILED DESCRIPTION

The present invention relates to a method of generating a score of a histopathological diagnosis of a cancer patient, comprising:

loading a first image patch into a processing unit, wherein the first image patch is cropped from a digital image of a slice of tissue, and wherein the slice of tissue has been immunohistochemically stained using a diagnostic antibody;

determining how many pixels of the first image patch belong to a first tissue that has been positively stained by the diagnostic antibody, wherein the determining is performed by processing the first image patch using a convolutional neural network;

processing additional image patches that have been cropped from the digital image to determine how many pixels of each additional image patch belong to the first tissue;

computing the score of the histopathological diagnosis based on a total number of pixels that belong to the first tissue; and

displaying the digital image and the score on a graphical user interface.

In a preferred embodiment, the determining how many pixels of the first image patch belong to the first tissue also involves determining how many pixels of the first image patch belong to a second tissue that has been negatively stained by the diagnostic antibody and to a third tissue that is neither the first tissue nor the second tissue.

In a preferred embodiment, the convolutional neural network is trained using generative adversarial learning.

In a preferred embodiment, the convolutional neural network is trained using a generative adversarial network that transforms image patches generated on a stain domain A into fake patches of a stain domain B.

In a preferred embodiment, the convolutional neural network is trained using a generative adversarial network that is trained to transform image patches generated on a stain domain A into fake patches of a stain domain B and then to perform segmentation on the stain domain B.

In a preferred embodiment, the convolutional neural network is trained using two generative adversarial networks, wherein a first of the two generative adversarial networks transforms image patches generated on a stain domain A into fake patches of a stain domain B, and wherein a second of the two generative adversarial networks transforms image patches generated on the stain domain B into fake patches of the stain domain A.

In a preferred embodiment, the convolutional neural network is trained using two generative adversarial networks, wherein a first of the two generative adversarial networks transforms image patches generated on a stain domain A into fake patches of a stain domain B and then performs segmentation on the stain domain B, and wherein a second of the two generative adversarial networks transforms image patches generated on the stain domain B into fake patches of the stain domain A and then performs segmentation on the stain domain A.

In a preferred embodiment, the slice of tissue is taken from a biopsy of lung cancer.

In a preferred embodiment, the diagnostic antibody is taken from the group consisting of: HER2, PD-L1, PD-L2, and CD73.

In a preferred embodiment, the score is a ratio of the total number of pixels that belong to the first tissue to a total number of pixels that belong to either the first tissue or the second tissue.

In a preferred embodiment, the score is a ratio of the total number of pixels that belong to the first tissue to a total number of pixels that belong to a selected tissue type, and wherein the selected tissue type is taken from the group consisting of: the first tissue, the second tissue and the third tissue.

In a preferred embodiment, the diagnostic antibody targets a protein, further comprising:
selecting a therapy if the score obtained using the diagnostic antibody is larger than a predetermined threshold, wherein the therapy that is selected uses a therapeutic antibody that binds to the protein targeted by the diagnostic antibody.

In a preferred embodiment, the diagnostic antibody is PD-L1, and wherein the selected therapy uses a PD-L1 medication if the score is larger than the predetermined threshold.

In a preferred embodiment, the first tissue that has been positively stained by the diagnostic antibody includes cells taken from the group consisting of: epithelial cells, endothelial cells, stromal cells, macrophages, T cells and B cells.

The present invention further relates to a method of generating a score of a histopathological diagnosis of a cancer patient, comprising:

loading an image patch into a processing unit, wherein the image patch is cropped from a digital image of a slice of tissue, and wherein the slice of tissue has been immunohistochemically stained using a diagnostic antibody;

performing image processing on the image patch using a generative adversarial network to train a convolutional neural network to determine how many pixels of the image patch belong to (a) a first tissue that has been positively stained by the diagnostic antibody, (b) a second tissue that has been negatively stained by the diagnostic antibody, or (c) a third tissue that is neither the first tissue nor the second tissue;

computing the score of the histopathological diagnosis based on a total number of pixels that belong to the first tissue; and

displaying the score on a graphical user interface.

In a preferred embodiment, the slice of tissue is lung tissue.

In a preferred embodiment, the diagnostic antibody is taken from the group consisting of: HER2, PD-L1, PD-L2, and CD73.

In a preferred embodiment, the convolutional neural network is trained using a cycle of two generative adversarial networks that jointly perform domain adaptation and segmentation.

In a preferred embodiment, the score is a ratio of the total number of pixels that belong to the first tissue to a total number of pixels that belong to the second tissue.

In a preferred embodiment, the method further comprises:
recommending a therapy for the cancer patient that uses a therapeutic antibody that targets the same protein as that targeted by the diagnostic antibody if the score obtained using the diagnostic antibody is larger than a predetermined threshold.

In a preferred embodiment, the diagnostic antibody is PD-L1, and wherein the therapy uses an anti-PD-L1 drug.

The present invention further relates to a system that generates a histopathological diagnosis for a cancer patient, comprising:

code for loading an image patch into a processing unit, wherein the image patch is cropped from an image of a tissue slice, and wherein the image was acquired by scanning cancer tissue that was immunohistochemically stained using a diagnostic antibody;

code for processing the image patch using a convolutional neural network to determine whether each pixel of the image patch belongs to (a) a tissue positively stained by the diagnostic antibody, (b) a tissue negatively stained by the diagnostic antibody, or (c) other tissue;

code for processing multiple image patches cropped from the image so as to compute a score for the histopathological diagnosis based on a total number of pixels determined to belong to the tissue that is positively stained by the diagnostic antibody; and

a graphical user interface that displays the image and the score.

In a preferred embodiment, the convolutional neural network is trained using a cycle of two generative adversarial networks that jointly perform domain adaptation and segmentation.

In a preferred embodiment, if the score is larger than a predetermined threshold, then the system recommends a therapy that uses a therapeutic antibody that targets the same protein as that targeted by the diagnostic antibody.

In a preferred embodiment, the diagnostic antibody is PD-L1, and wherein the therapy uses a drug targeting a protein of the group consisting of PD L1, PD-1.

Reference will now be made in detail to some embodiments of the invention, examples of which are illustrated in the accompanying drawings.

Example 1

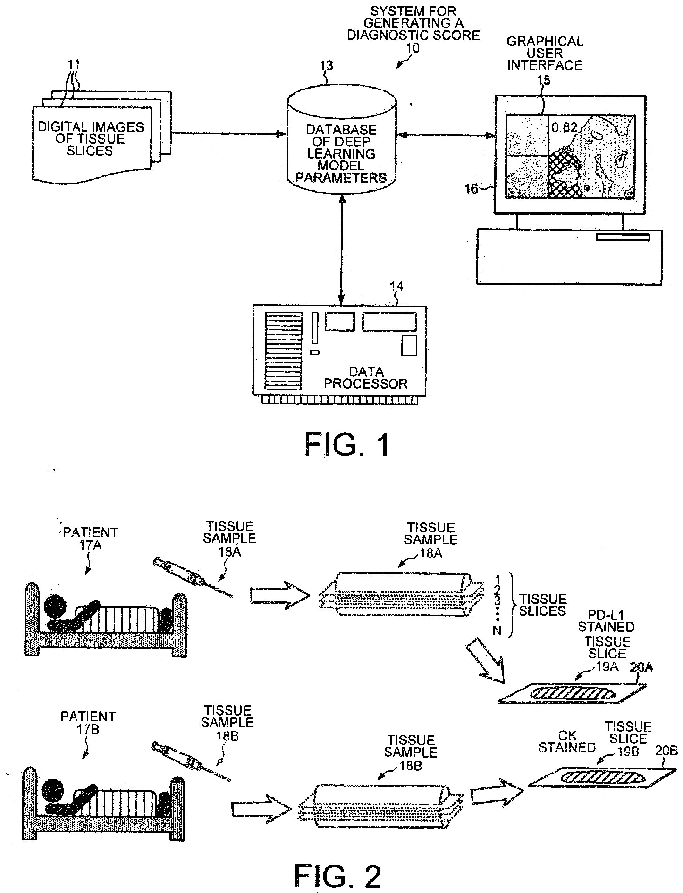

FIG. 1 shows a system 10 for generating a histopathological score for a cancer patient by computing a score for the histopathological diagnosis based on a total number of pixels determined to belong to tumor epithelial tissue that is positively stained by the diagnostic antibody. For example, the histopathological score is the Tumor Cell (TC) score. In one embodiment, the diagnostic antibody binds to the programmed death ligand 1 (PD-L1), and system 10 calculates a histopathological score for the cancer patient based on the total number of pixels of a digital image that have been determined to belong to tumor epithelium and to have been positively stained using the PD-L1 antibody. A high concentration of PD-L1 and thus a greater number of pixels determined to belong to tumor epithelium tissue that is positively stained by the PD-L1 antibody in solid tumors is indicative of a positive prognosis for patients treated by a PD-1/PD-L1 checkpoint inhibitor. Thus, system 10 analyzes digital images 11 to determine the total number of pixels that belong to first tissue that is positively stained using the diagnostic antibody PD-L1 and that is tumor epithelium. The first tissue is a specific group of tumor epithelial cells that are positively stained by the diagnostic antibody, in this embodiment by the PD-L1 antibody.

System 10 also identifies tissue that has been negatively stained by the diagnostic antibody. Tissue is considered to be "negatively stained" if the tissue is not positively stained by the diagnostic antibody. In this embodiment, the negatively stained tissue is tumor epithelial tissue that has not been positively stained by the PD-L1 antibody. The second tissue is a specific group of tumor epithelial cells that is negatively stained by the diagnostic antibody, in this embodiment by the PD-L1 antibody. In one embodiment, the first and second tissues that are positively and negatively stained by the diagnostic antibody belong to the same group of tumor epithelial cells. System 10 also identifies other tissue that belongs to different types of cells than the first and second tissues. The other tissue can be immune cells, necrotic cells, or any other cell type that is not the first or second tissue. The histopathological score computed by system 10 is displayed on a graphical user interface 15 of a user work station 16.

System 10 includes a convolutional neural network used for processing digital images and computing a score for the histopathological diagnosis of a cancer patient. The convolutional neural network is trained, wherein the training calculations are performed by a data processor 14. In one embodiment, data processor 14 is a specialized processor that can simultaneously perform multiple convolution operations between a plurality of pixel matrices and corresponding kernels. The logical operations of the model are implemented on data processor 14 as hardware, firmware, software, and/or a combination thereof to provide a means for characterizing regions of tissue in the digital image. Each trained model comprising an optimized set of parameters and associated mathematical operations is then stored in the database 13.

Once trained, system 10 reliably and precisely determines the total number of pixels that belong to tumor epithelium and that have been positively stained by the diagnostic antibody PD-L1. Training the convolutional neural network of system 10 by using a generative adversarial network obviates the need for extensive manual annotations of the digital images 11 that make up the training data set by transferring semi-automatically generated annotations on digital images of tissue stained with the epithelial cell marker CK to the PD-L1 domain. The biomarker CK specifically labels tumor epithelium, thereby allowing for a semi-automated segmentation of tumor epithelial regions based on the CK staining. After semantic segmentation, the digital images of tissue stained with the epithelial cell marker CK are transformed into the PD-LI domain. During this step, synthetic or fake images are generated. The regions identified as epithelial cells (positive for CK staining) are labeled as being either positive for PD-L1 staining (PD-L1 expressing cells) or negative for PD-L1 staining (non-PD-L1 expressing cells). The resulting fake images of tissue stained with PD-L1 antibody that are generated based on the images of tissue stained using CK are then used in conjunction with a reduced number of images with manual annotations to train the convolutional neural network of system 10 to identify positively stained tissue in digital images of tissue stained with the PD-L1 antibody.

FIG. 2 illustrates tissue samples 18A and 18B being taken from cancer patients 17A and 17B, respectively. A tissue slice 19A from tissue sample 18A is placed on a slide 20A and stained with a diagnostic antibody, such as the PD-L1 antibody. The tissue slice 19B is placed on the slide 20B and stained with a helper antibody, such as the CK antibody. The helper antibody is used only to train system 10, whereas the diagnostic antibody is used to compute the histopathological score in a clinical application of system 10. High resolution digital images are acquired from slice 19A and slice 19B of the tissue from cancer patients 17A and 17B. The tissue samples 18A and 18B can be taken from a patient suffering from non-small-cell lung cancer, other types of lung cancer, breast cancer, prostate cancer, pancreatic cancer or another type of cancer. In one embodiment, the tissue sample 18A is taken from a solid tumor. In another embodiment, the tissue sample has been stained with a PD-L1 antibody, and in yet another embodiment with an HER2 antibody. In one embodiment, regions comprising normal epithelial cells (as opposed to tumor epithelium) are excluded from the digital images acquired from slice 19A and slice 19B.

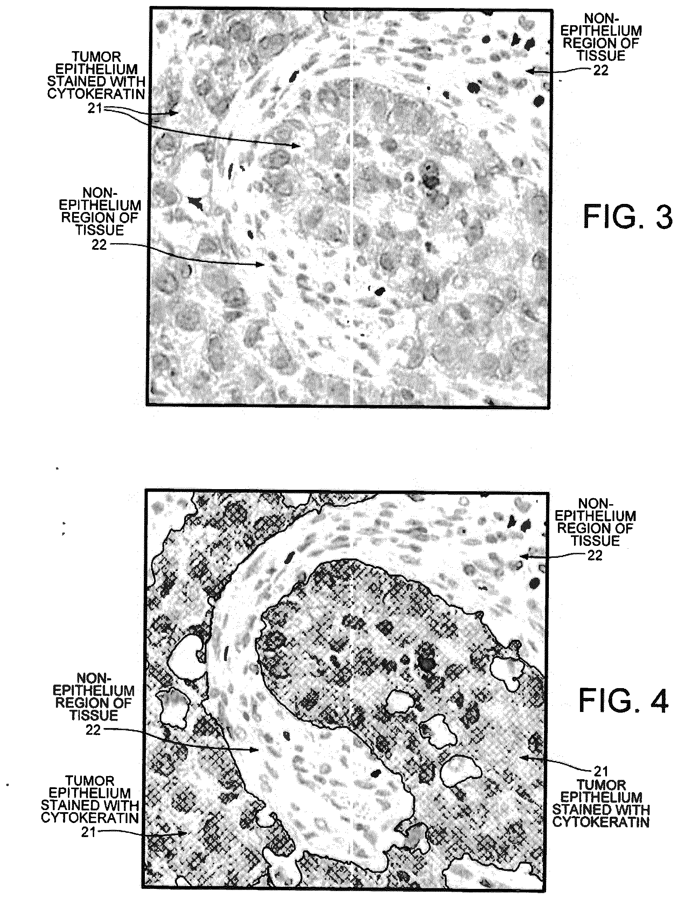

FIG. 3 shows a digital image acquired from slice 19B of the tissue from cancer patient 17B that has been stained using the CK antibody. Epithelial cells 21 are stained positive by the CK stain. Non-epithelial region 22 is not stained positively by the CK stain. The identification and segmentation of CK-positive epithelial cells and non- CK-positive other tissue is a first substep of the overall task of identifying epithelial cells that are positively stained using PD-L1.

FIG. 4 shows the digital image of FIG. 3 in which the regions have been identified and segmented. The region 21 corresponding to CK-positively stained tissue has been marked with cross hatching and corresponds to tumor epithelium.

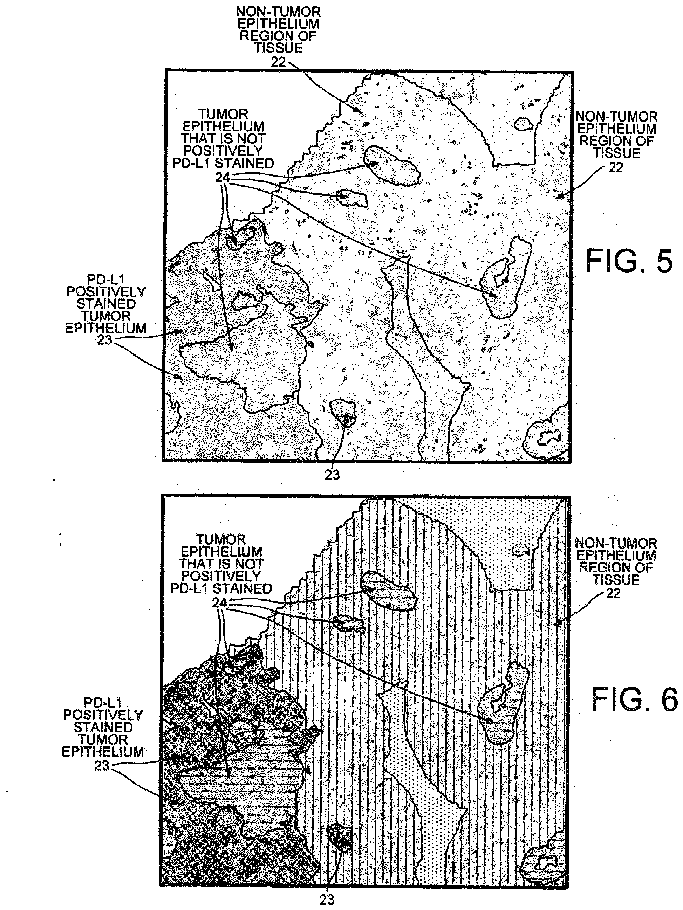

FIG. 5 shows a digital image acquired from slice 19A of the tissue from cancer patient 17A that has been stained for the PD-L1 antibody. The digital image has been segmented into regions corresponding to regions 23 of tumor epithelial cells positively stained using the PD-L1 antibody, regions 24 of tumor epithelial cells that are not positively stained by the PD-L1 antibody, and regions 22 of other tissue (e.g., immune cells such as macrophages, necrotic tissue etc.). System 10 uses a convolutional neural network to determine for each pixel of the digital image whether that pixel belongs to tumor epithelial tissue positively stained by the PD-L1 antibody and thus belonging to region 23, tissue negatively (non-positively) stained by the PD-L1 antibody and thus belonging to region 24, or other tissue belonging to region 22. The score for the histopathological prognosis is calculated by dividing the total number of pixels determined to belong to the tumor epithelial tissue that is positively stained by the diagnostic antibody PD-L1 by the total number of pixels determined to belong both to the tumor epithelial tissue that is positively stained by the diagnostic antibody PD-L1 as well as to the tumor epithelial tissue that is not positively stained by the diagnostic antibody PD-L1 (regions 23 and 24).

FIG. 6 shows the image of FIG. 5 segmented into the regions 22, 23 and 24. The region 23 corresponding to PD-L1-positively stained tumor epithelium is marked with cross hatching. The region 24 of tumor epithelium that is not PD-L1-positively stained is marked with horizontal lines. The region 22 that is non-epithelial tissue is marked with vertical lines.

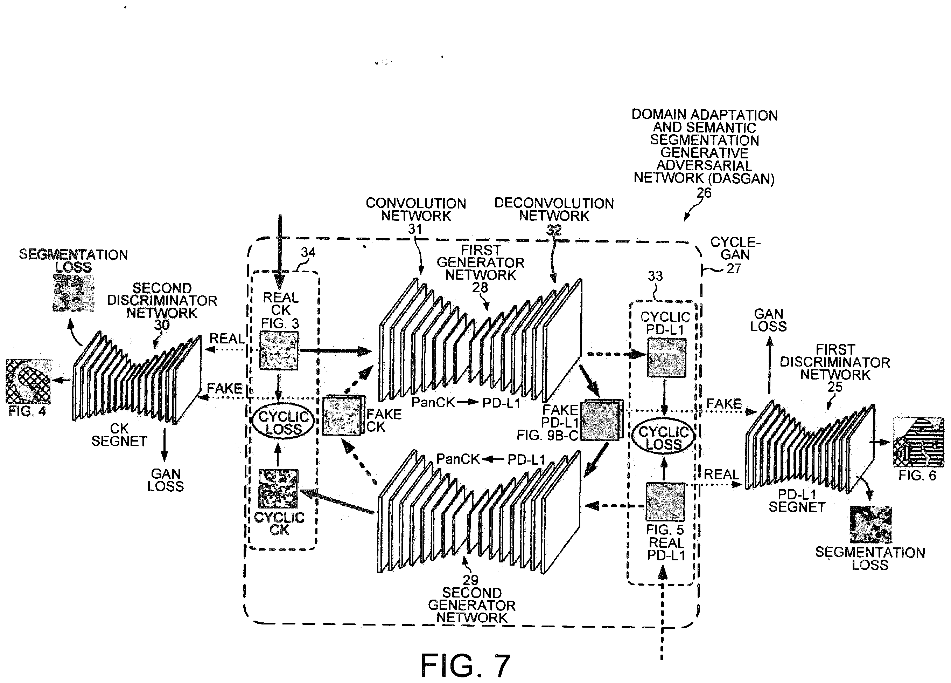

FIG. 7 illustrates a convolutional neural network 25 used in a clinical application for determining how many pixels of a first image patch belong to a first tissue that has been positively stained by the diagnostic antibody PD-L1 and that belongs to tumor epithelium, and a generative adversarial network 26 used for training the convolutional neural network 25. The convolutional neural network 25 acts as a discriminator in the framework of the domain adaptation and semantic segmentation generative adversarial network (DASGAN) 26. In one embodiment, the DASGAN includes two generative adversarial networks that operate as a cycle forming a CycleGAN 27. The tissue slides stained with CK and PD-L1 that are used to train network 25 need not be from tissue of the same patient. Thus, FIG. 2 shows the PD-L1-stained tissue slice 19A coming from patient 17A and the CK-stained tissue slice 19B coming from patient 17B. When network 25 is deployed to generate a score, only the images of tissue slices stained by the diagnostic antibody, such as PD-L1, are used. Images of tissue slices stained by the helper antibody, such as CK, are used only for training the algorithm of the convolutional neural network 25. Network 25 is trained to recognize pixels of a first tissue that is associated with both fake CK positive staining and real PD-L1 positive staining.

The CycleGAN 27 includes two generative adversarial networks. The first of the two generative adversarial networks includes a first generator network 28 and a first discriminator network 25. The first generator network 28 transforms image patches generated on CK-stained tissue slices into fake patches of digital images of PD-L1 stained tissue slices. The first discriminator network 25 learns to distinguish digital images of real PD-L1 stained tissue slices from the fake images of PD-L1 stained tissue slices generated by the first generator network 28 and segments the PD-L1 positive and the PD-L1 negative regions in the digital images. The first generator network 28 transforms image patches generated on the stain domain of CK into fake patches of the stain domain of PD-L1. In one embodiment, the first discriminator network 25 is used to determine how many pixels of an image patch belong to tumor epithelium tissue that has been positively stained by a diagnostic antibody, for example, by the PD-L1 antibody.

The second of the two generative adversarial networks includes a second generator network 29 and a second discriminator network 30. The second generator network 29 transforms image patches generated on PD-LI-stained tissue slices into fake patches of digital images of CK stained tissue slices. The second discriminator network 30 learns to distinguish digital images of real CK-stained tissue slices from the fake images of CK-stained tissue slices generated by the second generator network 29 and segments the CK positive and the CK negative regions in the digital images. The second generator network 29 transforms image patches generated on the stain domain of PD-L1 into fake patches of the stain domain of CK.

The first generator network 28, the second generator network 29, the first discriminator network 25 and the second discriminator network 30 each contain a convolution network and a deconvolution network that perform several layers of convolution and deconvolution steps, respectively. For example, the first generator network 28 includes a convolution network 31 and a deconvolution network 32.

The fake images of PD-L.1 stained tissue slices generated by the first generator network 28 together with real images of PD-L1 stained tissue slices are fed into the second generator network 29 that generates fake images of CK stained tissue slices. The fake images of CK stained tissue slices generated by the second generator network 29 together with real images of CK stained tissue slices are fed into the first generator network 28 that generates fake images of PD-L1 stained tissue slices. For example, network 28 generates first fake images of tumor epithelium positively stained with the PD-L1 antibody as well as second fake images of PD-L1 negatively stained tumor epithelium.

To ensure the invertibility of the transformed domains (e.g., the domain of CK staining and the domain of PD-L1 staining), the CycleGAN 27 also includes cycle consistency losses 33-34. The network 28 transforms the fake CK images that have been generated by the network 29 from tissue slices that have been stained with PD-L1 back into the PD-L1 stain domain, thereby creating fake PD-L1 images from the fake CK images. The fake PD-L1 images generated by successively applying the generator network 29 and the generator network 28 are also referred to as "cyclic PD-L1" in FIG. 7. The network 29 transforms the fake PD-L1 images that have been generated by the network 28 from tissue slices that have been stained with CK back into the CK stain domain, thereby creating fake CK images from the fake PD-L1 images. The fake CK images generated by successively applying the generator network 28 and the generator network 29 are also referred to as "cyclic CK" in FIG. 7.

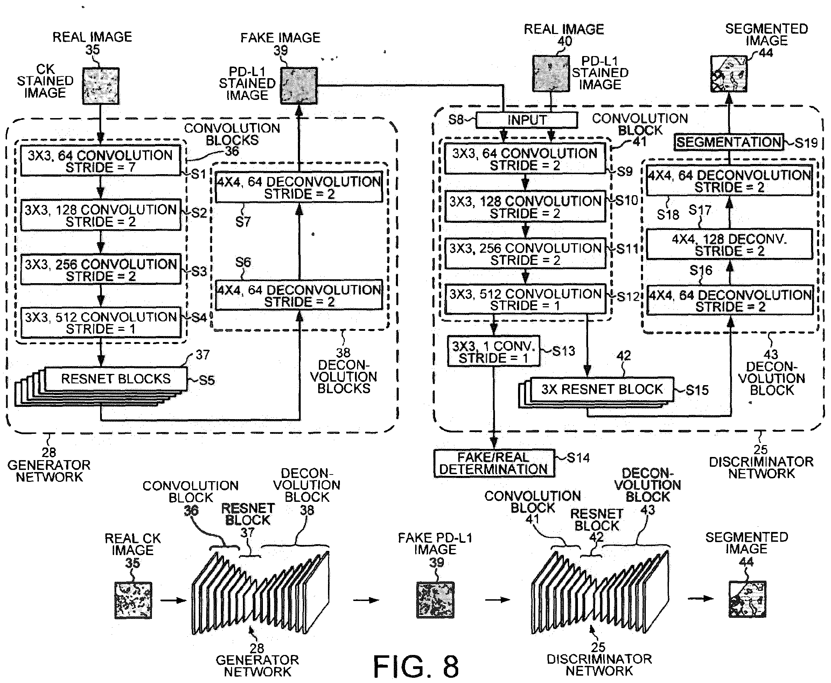

FIG. 8 illustrates the first generator network 28 and the first discriminator network 25 of FIG. 7 in more detail. The first generator network 28 and the first discriminator network 25 form one of the generative adversarial networks of the CycleGAN 27. A real image 35, in this example a digital image of a CK-stained tissue slice, is input into the first generator network 28. The first generator network 28 includes a series of convolution blocks 36, of a series of residual network (ResNet) blocks 37 and of a series of deconvolution blocks 38. First generator network 28 performs several convolution, normalization and activation steps in convolution blocks 36. In a step S1, a convolution operation is carried out using a 3X3 operator with a stride of seven giving rise to a pixel patch with sixty-four convolution layers. In step S2, convolution is carried out using a 3x3 operator with a stride of two giving rise to pixels with 128 feature layers. In step S3, convolution is carried out on the 128 feature layers using a 3x3 operator with a stride of two giving rise to pixels with 256 feature layers. In step S4, convolution is carried out on the 256 feature layers using a 3x3 operator with a stride of one giving rise to pixels with 512 feature layers. Each convolution step is followed by an instance normalization step and a rectified linear unit (ReLu) activation step.

In step S5, six ResNet blocks 37 are applied before deconvolution commences in deconvolution block 38. The operations of the ResNet block 37 enable deep-layered networks to be trained with less computational burden on the network processing by allowing the information flow to bypass selected layers of the network. In step S6, deconvolution is carried out using a 4x4 operator with a stride of two giving rise to pixels with 64 feature layers. In step S7, deconvolution is carried out using a 4x4 operator with a stride of two giving rise to pixels with 64 feature layers. In step S7, deconvolution block 38 generates a fake image 39 in a different stain domain, in this example in the new domain of PD-L1 staining.

The first discriminator network 25 is then trained to segment PL-L1 images, whether fake or real, and to classify individual pixels as being stained by PL-L1 by using the fake PD-L1 images 39 generated by generator network 28 along with real PD-L1 images 40. In step S8, the fake image 39 output in step S7 is input into the first discriminator network 25 together with the real image 40 of the same stain domain, for example, a digital image that has been acquired from a tissue slice stained with PD-L1 antibody. For example, the first discriminator network 25 can be trained based on the fake PD-L1 images generated by the first generator network 28 and the associated groundtruth masks. The fake PD-L1 images generated by the first generator network 28 are then used for training in conjunction with manual annotations on real PD-L1 images acquired from tissue slices stained with PD-L1 antibody. In one embodiment, the complete DASGAN network 26, consisting of the network 27 (CycleGAN) and of the two SegNet networks 25 (PD-L1 SegNet) and 30 (CK SegNet) are trained simultaneously. Although "simultaneous" training still involves sequential steps on a computer, the individual optimizing steps of training both networks are interwoven.

The first discriminator network includes a convolution block 41, a ResNet block 42 and a deconvolution block 43. Several convolution, normalization and activation steps are performed in the convolution block 41. In step S9, convolution is carried out using a 3x3 operator with a stride of two giving rise to pixels with 64 feature layers. In step S10, convolution is carried out using a 3x3 operator with a stride of two giving rise to pixels with 128 feature layers. In step S11, convolution is carried out using a 3x3 operator with a stride of 2 giving rise to pixels with 256 feature layers. Each convolution step is followed by an instance normalization step and a rectified linear unit (ReLu) activation step. In step S13, convolution is carried out using a 3x3 operator with a stride of one giving rise to pixels with one feature layer. In step S14, the first discriminator network 25 determines the source of the input data, i.e., whether the image input into the discriminator 25 was a fake image of an PD-L1 staining generated by the generator 28 based on images of CK-stained tissue slices or a real image acquired from a tissue slice stained by PD-L1 antibody.

In step S15, three ResNet blocks 42 are applied to the convoluted image output by convolution block 41 in step S12. Then deconvolution block 43 performs deconvolution operations on the output of the ResNet blocks 42. In step S16, deconvolution is carried out using a 4x4 operator with a stride of 2 giving rise to pixels with 64 convolution layers. In step S17, deconvolution is carried out using a 4x4 operator with a stride of two giving rise to pixels with 128 layers. In step S18, deconvolution is carried out using a 4x4 operator with a stride of two giving rise to pixels with 64 layers. In step S19, the first discriminator network 25 performs segmentation to determine which pixels of the image patch belong to the first tissue that has been identified as positively stained for PD-L1. The classification as being positively stained for PD-L1 is performed on a pixel-by-pixel basis. The first discriminator network 25 also determines which pixels of the image patch belong to the second tissue that has been identified as not being positively stained (also known as being "negatively stained") for PD-L1.

The first discriminator network 25 can a_so determine which pixels of the image patch belong to other tissue that corresponds to a different group of cells or cell type other than the first tissue or the second tissue. For example, the first tissue and second tissue can both be epithelial cells, and the other tissue can be immune cells.

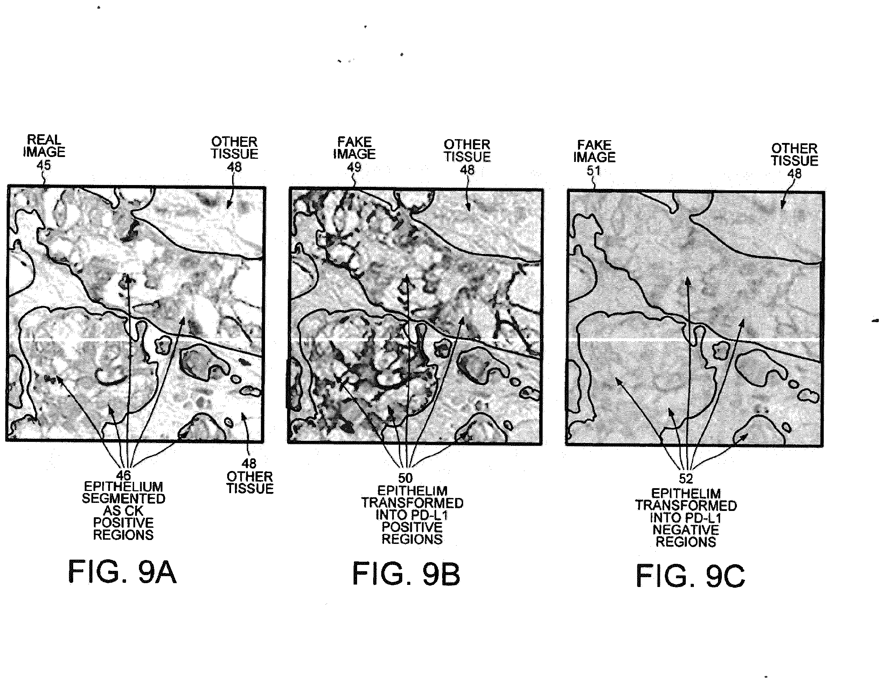

FIG. 9A shows a real digital image 45 of a tissue slice stained with CK, with segmented regions of CK-positive epithelial cells. Because of the specificity of the CK biomarker to epithelium, epithelium regions are segmented on CK stained images using heuristic-based image analysis, for example color deconvolution followed by Otsu thresholding and morphological operations. Any remaining false positive detections are removed by manual annotation. In one example, the digital image 45 has 256x256 pixels. Epithelial cells are marked with the reference numeral 46, and non-epithelial tissue is marked with the reference numeral 48. Segmented images of tissue slices stained with CK are then input into the first generator network 28, which transforms the images of tissue slices stained for CK into synthetic or fake images corresponding to tissue stained with the PD-L1 antibody. The first generator network 28 thus performs image-to-image translation.

FIG. 9B shows a fake or synthetic image 49 of the PD-L1 antibody generated to be PD-L1 positive by the generator 28 from the real image 45 of FIG. 9A, with segmented regions 50 of PD-L1 positive cells generated from the segmented regions 46 of epithelium cells of FIG. 9A. Pixels in the regions of epithelium cells 46 in FIG. 9A are generated to be positively stained by PD-L1 in FIG. 9B, yielding the regions 50 to be classified as positively stained with PD-L1.

FIG. 9C shows a fake or synthetic image 51 of the PD-L1 antibody generated to be PD-L1 negative by the generator 28 from the real image 45 of FIG. 9A, with segmented regions 52 of PD-L1 negative cells generated from the segmented regions 46 of epithelium cells of FIG. 9A. Pixels in the regions of epithelium cells 46 in FIG. 9A are generated to be negatively stained by PD-L1 in FIG. 9C, yielding the regions 52 to be classified as negatively stained with PD-L1. Regions of fake image 51 are synthesized as PD-L1 negative using the knowledge of which cells were positively stained with CK in FIG. 9A combined with the trained deep learning knowledge of how the CK staining would correspond to negative PD-L1 staining. The fake images 49 and 51 have 128x128 pixels each. The first generator network 28 performs a "domain adaptation" from the CK stain domain to the PD-L1 stain domain by using segmented input images of CK-stained tissue.

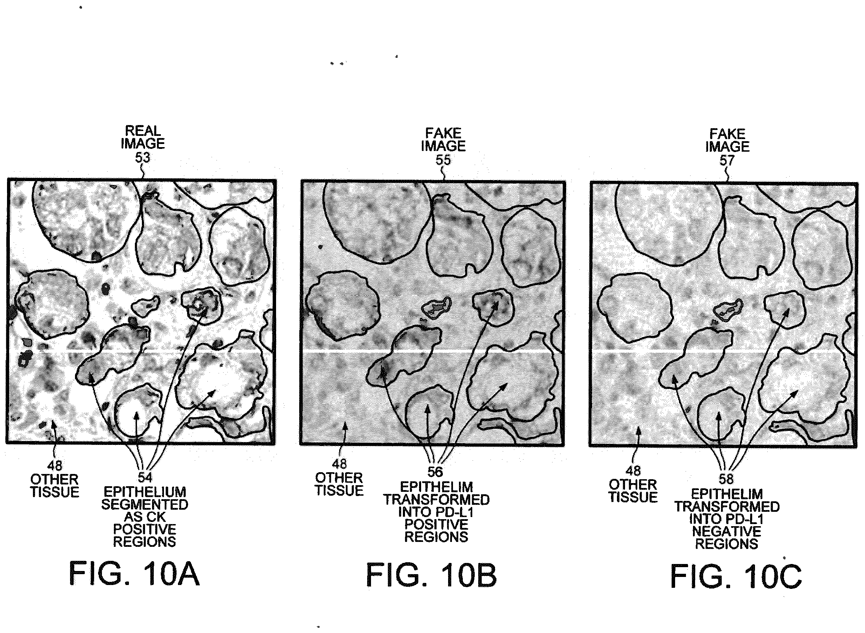

FIG. 10A shows another example of a real digital image 53 of a tissue slice stained for CK, with segmented regions of CK-positive epithelial cells. Real image 53 has smaller regions of contiguous CK-stained epithelial cells than does real image 45 of FIG. 9A. Image 53 shows epithelial cells 54 stained positively for CK, and other tissue 48 that is not epithelial cells 54. These segmented images of tissue slices stained for CK are then input into the first generator network 28 that transforms the images of tissue slices stained for CK into fake images of tissue slices stained with the PD-L1 antibody.

FIG. 10B shows a fake or synthetic image 55 of the PD-L1 antibody generated to be PD-L1 positive by the generator 28 from the real image 53 of FIG. 10A, with segmented regions 56 of PD-L1 positive cells generated from the segmented regions 54 of epithelium cells of FIG. 10A. Pixels in the regions of epithelium cells 54 in FIG. 10A are generated to be positively stained by PD-L1 in FIG. 10B, yielding the regions 56 to be classified as positively stained with PD-L1. FIG. 10C shows a fake or synthetic image 57 of the PD-L1 antibody generated to be PD-L1 negative by the generator 28 from the real image 53 of FIG. 10A, with segmented regions 58 of PD-L1 negative cells generated from the segmented regions 54 of epithelium cells of FIG. 10A. Pixels in the regions of epithelium cells 54 in FIG. 10A are generated to be negatively stained by PD-L1 in FIG. 10B, yielding the regions 57 to be classified as negatively stained with PD-L1.

The stain used to make the real images operated upon by DASGAN 26 can be an antibody with a dye or a direct stain. The antibody can be a polyclonal antibody or a monoclonal antibody and can be directly conjugated with an entity allowing detection of the antibody or can be detected by use of a secondary antibody conjugated with an entity allowing detection of the antibody. The first tissue that has been positively stained by the diagnostic antibody includes tumor epithelial cell types. Examples of the diagnostic antibody include PD-L1, human epidermal growth factor receptor 2 (HER2), PD-L2, CTLA4 and CD73 antibodies. In other embodiments, the scoring is performed on cell types other than the CK positive epithelial cells. In those applications, the CK antibody is replaced by other celltype-specific antibodies, such as CD20 for B-cells, CD34 for endothelial cells, CD3 for T-cells and CD68 for macrophages.

In order to train the first discriminator network 25, fake images of tissue slices stained with the PD-L1 antibody, which are generated by the first generator network 28, are input alongside real images of tissue slices stained with the PD-L1 antibody into the first discriminator network 25 at step S8 of FIG. 8. The first discriminator network 25 is trained to distinguish in step S14 the fake images of tissue slices stained with the PD-L1 antibody as generated by the first generator network 28 from the real images of tissue slices stained with the PD-L1 antibody. The first discriminator network 25 is also trained to perform segmentation on the PD-L1 images, both fake and real, to identify which pixels of the images belong to PD-L1 positive epithelial cells. Each pixel of the segmented image 44 of FIG. 8 is classified as belonging to a PD-L1 positively stained epithelial cell (the first tissue), an epithelial cell that is not positively stained by PD-L1, or other non-epithelial tissue. Segmentation maps are thus obtained as an output of the first discriminator network 25 in addition to the determination of whether an image is real or fake. The second discriminator network 30 performs the same operations on real and fake images in the CK stain domain.

In one embodiment, the first discriminator network 25 is deployed as a convolutional network to carry out the step of determining how many pixels of a first image patch belong to a first tissue that has been positively stained by the diagnostic antibody and that belongs to tumor epithelium. For example, the first tissue corresponds to tumor epithelial cells, and the diagnostic antibody is the PD-L1 antibody. In this case, the convolutional neural network determines which pixels of the first image patch belong to epithelial cells positively stained by the PD-L1 antibody.

Subsequently, a score of the histopathological diagnosis of the patient 17 is computed based on the total number of pixels of the image patch that have been determined to belong to a first tissue. For example, the score can be the Tumor Cell (TC) score. In one embodiment, the method involves selecting a therapy if the score obtained using the diagnostic antibody is larger than a predetermined threshold. The therapy that is selected uses a therapeutic antibody that binds to the protein targeted by the diagnostic antibody, e.g., to the PD-L1 protein targeted by the diagnostic antibody PD-L1.

For example, if a score of 0.6 is computed, this is indicative of 60% of the pixels in the image patch belonging to tumor epithelial tissue that has been positively stained by the PD-L1 antibody. The score is computed as the percentage of tumor epithelial cells that are PD-L1 positive. The score is calculated as the ratio of the total number of pixels that belong to the first tissue (e.g., tumor epithelial cells positively stained by the PD-L1 antibody) in relation to the total number of pixels that belong to the first tissue plus the second tissue (e.g., all tumor epithelial cells, including positively stained by the PD-L1 antibody as well as not positively stained by the PD-L1 antibody). In other words, a score of 0.6 indicates that 60% of the tumor epithelial cells in the tissue slice from which the digital image was acquired express the PD-L1 protein.

Evaluation of the score is performed using the predetermined threshold. For example, if the threshold of the score is set to 0.5, the computed score of 0.6 is above the threshold. In that case, a therapy is selected that uses a therapeutic antibody that binds to the protein (e.g., PD-L1) targeted by the diagnostic antibody (PD-L1). In this example, a PD1/PD-L1 check point inhibitor therapy would be selected.

In one example, the score is calculated as follows: the number of pixels in an image determined as belonging to the first tissue stained positively for the diagnostic antibody and belonging to tumor epithelium (e.g., PD-L1 positive epithelial cells) is 4,648,680. The number of pixels determined to correspond to the second tissue that belongs to tumor epithelium but that is not stained positively by the diagnostic antibody (e.g., PD-L1 negative epithelial cells) is 1,020,158. The Tumor Cell (TC) score is then calculated to be 0.820, which equals 4648680/(4648680+1020158).

Although the present invention has been described in connection with certain specific embodiments for instructional purposes, the present invention is not limited thereto. Various modifications, adaptations, and combinations of various features of the described embodiments can be practiced without departing from the scope of the invention as set forth in the claims.

Example 2

DASGAN - Joint Domain Adaptation and Segmentation for the Analysis of Epithelial Regions in Histopathology PD-L1 Images

The automated analysis of the tumor microenvironment on digital histopathology slides is becoming key for the understanding of the immune response against cancer, supporting the development of novel immuno-therapies. The inventors propose a novel deep learning solution to the related problem of tumor epithelial region detection. While most existing deep learning segmentation approaches are trained on time-consuming and costly manual annotation on single stain domain (PD-L1), the inventors leverage here semi-automatically labeled images from a second stain domain (Cytokeratin). The inventors introduce an end-to-end trainable model that jointly performs image-to-image translation between the two stain domains while segmenting tumor epithelium. Extending the method to differentiate between the PD-L1 positive and negative tumor epithelium regions enables the automated estimation of the PD-L1 Tumor Cell (TC) score. Quantitative experimental results demonstrate the accuracy of the inventors' approach against state-of-the-art segmentation methods.

1. Introduction

Histopathology is key to many clinical decisions taken in oncology, based on the visual quantification of biomarkers on stained slides of suspected tumor tissue. In a clinical setting, the PD-L1 Tumor Cell (TC) score for Non Small Cell Lung Cancer (NSCLC) is for instance predictive of response for patients treated with an anti-PD1/PD-L1 checkpoint inhibitor therapy. In a more exploratory setting, several studies have shown the importance of tumor immune contexture and of epithelial immune cell infiltration as measures of patient prognosis.

While different from the PD-L1 scoring setting, these applications also depend on the precise estimation of the tumor epithelial compartment. These common problems lead us to introduce a novel method for the automatic and precise segmentation of epithelium in histopathology images.

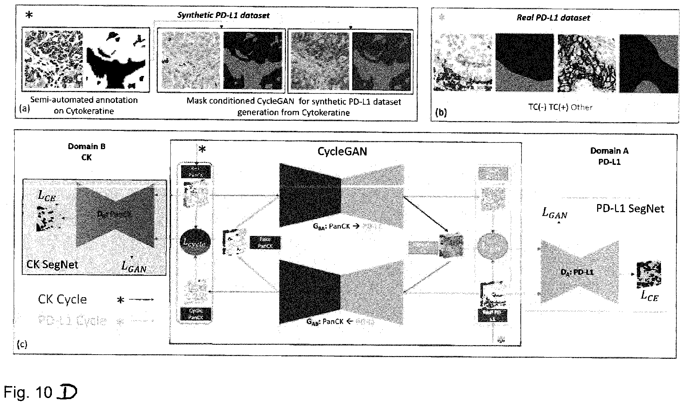

As illustrated in Fig. 10D (b), a key challenge is the non-specificity of PD-L1 staining, which includes tumor epithelial regions as well as immune cells and necrotic regions. The performance of deep learning approaches for histopathology image analysis leads us towards this field. Previous attempts at TC scoring are based on patchclassification and are restricted by their low resolution segmentation output. Moving towards pixelwise semantic segmentation leads to the challenge of generating large, time-consuming and costly datasets of boundary-precise manual annotations of tumor epithelium[12]. Here, the inventors lower the annotation burden by transferring semi-automatically generated annotations on slides stained with the well-established epithelial marker cytokeratin (CK) (Fig. 10D (a)) to the PD-L1 stain domain. CK specifically labels tumor epithelium, which makes a semi-automated segmentation of epithelial regions in this domain possible using coarse manual input combined with simple heuristic rules. This enables the generation of large and boundary-precise datasets for semantic segmentation. After transfer to the PD-L1 stain domain, the resulting synthetic CK-based dataset is used for training in conjunction with manual annotations on true PD-L1 images.

The inventors synthesize fake but realistic looking PD-L1 images from real CK images by exploiting recent advances of generative adversarial networks (GANs) and in particular of image-to-image translation. Unpaired image-to-image translation using CycleGAN makes the transformation of CK stained images into synthetic PD-L1 stained images possible. This enables the use of semi-automatically generated annotations from CK without the need for coregistration between consecutive or restained slides. In pathology image analysis, CycleGAN was first introduced for normalization of HE stained images. The inventors present here what is to their knowledge the first application of domain adaptation based semantic segmentation in this field. Other studies recently described similar ideas for medical image analysis, in particular to convert CT images into synthetic MRI data and train a semantic segmentation model on both real and synthetic MRI images. However, while these prior approaches follow a two-step methodology, the inventors introduce here an end-to-end trainable network (cf. Fig. 10D (c)) named DASGAN (Domain Adaptation and Segmentation GAN) that jointly performs image-to-image translation and semantic segmentation. The inventors show superiority of the proposed DASGAN model compared to (i) models trained on manual annotations on real PD-L1 images only and (ii) models trained separately for domain adaptation and for semantic segmentation.

## 2. Methods

The inventors' main contribution is the extension of the CycleGAN towards an end-to-end trainable network for joint domain adaptation and segmentation. While the proposed network enables merging annotations from any two arbitrary stain domains, the inventors specifically apply it to transfer from the CK stain domain to PD-L1 stain domain with the objective of limiting the burden of manual annotations for epithelium detection. The good specificity of CK helps in accurate segmentation of epithelial regions in CK stain domain using heuristic rules, more precisely color deconvolution followed by automatic thresholding and closing morphological operations (cf. Fig. 10D (a)). The following paragraphs detail how these CK-based annotations are leveraged in the PD-L1 stain domain.

## 2.1 CycleGAN

Two generators $G_{BA} : \mathcal{X}_B \to \mathcal{X}'_A$ and $G_{AB} : \mathcal{X}_A \to \mathcal{X}'_B$

are trained to synthesize samples in domain A (PD-L1) from real samples in domain B (CK) and vice versa. Two discriminators $D_A$ and $D_B$ are trained in opposition to identify synthetic from real samples in the two domains. The necessity of having image pairs for image translation between A and B can be bypassed using a cycle consistent loss $\mathcal{L}_{cycle}$. The parameters of the discriminator and generator networks are learned in an adversarial manner following a min-max game:

$$\min_{G_{AB}} \max_{D_B} \mathcal{L}_{GAN}^{AB} := \mathbb{E}_{x_B \sim \mathcal{X}_B} \log(D_B(x_B)) + \mathbb{E}_{x_A \sim \mathcal{X}_A} \log(1 - D_B(G_{AB}(x_A))) \quad (1)$$

$$\min_{G_{BA}} \max_{D_A} \mathcal{L}_{GAN}^{BA} := \mathbb{E}_{x_A \sim \mathcal{X}_A} \log(D_A(x_A)) + \mathbb{E}_{x_B \sim \mathcal{X}_B} \log(1 - D_A(G_{BA}(x_B))) \quad (2)$$

To avoid mode collapse and enable invertability of the translated domains, a cycle consistency loss is defined by re-translating the synthesized samples

$$x'_B = G_{AB}(x_A)$$

and

$$x'_A = G_{BA}(x_B)$$

back to their original domains A and B:

$$\mathcal{L}_{cycle} = \mathbb{E}_{x_A \sim \mathcal{X}_A} \|x_A - G_{BA}(x'_B)\| + \mathbb{E}_{x_B \sim \mathcal{X}_B} \|x_B - G_{AB}(x'_A)\| \qquad (3)$$

2.2 DASGAN

Following the auxiliary class GAN (AC-GAN) model, the inventors extend the Cycle-GAN model to obtain segmentation maps as auxiliary from the two discriminators $D_A$ and $D_B$ (cf. Fig. 10D (c)). The inventors condition the input images of $G_{AB}$ and $G_{BA}$ with the respective ground truth segmentation class mask by concatenating the mask across the input image channel axis. The respective concatenated volumes go through a series of transformations by $G_{AB}$ and $G_{BA}$ to produce synthetic images in the respective target domains B and A. The two discriminator networks are extended to predict pixel-wise class probability maps in addition to predicting the correct source of image. To this end, and to propagate the class specific information through to the generator, a segmentation loss is introduced to the discriminator in addition to the original adversarial loss:

$$\mathcal{L}_{seg} := \mathcal{L}_{CE}(y_A^{true}, y_A^{pred}) + \mathcal{L}_{CE}(y_B^{true}, y_B^{pred}) \qquad (4)$$

where

$$\mathcal{L}_{CE}(y^{true}, y^{pred}) = -\sum y^{true} \log(y^{pred})$$

denotes the categorical cross-entropy loss and where

$$y^{true}$$

and

$$y^{pred}$$

correspond to the ground truth and the predicted label maps respectively. This results in the following loss for the proposed joint domain adaptation and semantic segmentation DASGAN model:

$$\mathcal{L} := \mathcal{L}_{GAN}^{AB} + \mathcal{L}_{GAN}^{BA} + \lambda_1 \mathcal{L}_{cycle} + \lambda_2 \mathcal{L}_{seg}, \qquad (5)$$

with

$$\lambda_1 = 10$$

and

$$\lambda_2 = 1$$

weighting the losses associated with the cycle constrain and the segmentation auxiliary task respectively. The proposed DASGAN model is used to leverage annotations on CK stained images for the segmentation of epithelial regions in PD-L1 stained images. To additionally differentiate between positive and negative tumor epithelial regions, and therefore enable the calculation of the Tumor Cell score, the inventors perform three-class pixelwise mask conditioning and transform the tumor epithelial annotations obtained in the CK domain into both a negative and a positive tumor epithelial synthetic example in the PD-L1 domain (cf. Fig. 10D (a)).

### 3. Experiments and Results

After describing the train, test and validation datasets as well as the network architectures, the inventors present results of quantitative evaluation against pathologist manual annotations for the two problems of tumor epithelial segmentation and PD-L1 positive and negative tumor epithelial region detection.

### 3.1 Cytokeratin and PD-L1 Datasets

The training dataset consists of n = 56 CK stained images of NSCLC samples and of n = 69 whole slide images of the same indication stained with SP263 PD-L1 clone. To ensure purity of the samples generated on the CK stained slides, the samples are exclusively created on tumor center regions delineated by pathologists and regions with non-specific staining are discarded. Note that, because this manual input is typically provided at a macroscopic scale (1x or 2x), the associated effort is minimal compared to the annotation of fine epithelium structure at high resolution (20x). Positive (TC+) and negative (TC-) tumor epithelium regions were manually delineated at high resolution on PD-L1 images, together with non-epithelium regions (e.g. immune, necrotic, and stromal regions).

The manually annotated PD-L1 dataset is split between training, test and validation sets on the slide level. The validation set consists of n = 106 fields of view (500 x 500 $\mu$m) densely annotated by pathologists and taken from 25 PD-L1 stained whole slide images. The validation set was chosen to cover a high variability of different cancer types (adeno, squamous) and growth patterns (acinar, papillary and solid). While the validation dataset used for quantitative evaluation remains unchanged, and in order to study the effect of increasing availability of labeled images and patches for training, three different configurations are reported for the training set: (i) 44K patches from 22 slides, (ii) 103K patches from 49 slides and (iii) 149K patches from 69 slides. The test set, used for model selection, is constant and consists of 16K patches from 8 PD-L1 stained slides.

Patches of 128 x 128 pixels were sampled at a uniform grid from the manually annotated regions on the PD-L1 stained slides and from the detected epithelium regions on the CK stained slides at a 10x magnification (1 $\mu$m/px).

### 3.2 Network architectures

The architectures of the two generators are similar to that in the original Cycle-GAN with minor modifications: the inventors concatenate the input images and the segmentation mask. For the two discriminators, weights between the prediction of the source distribution and of the semantic segmentation posterior maps are shared in the first three convolutional layers and the branch for semantic segmentation extended to include three resnet blocks and three deconvolutional layers. Spectral normalization and self-attention blocks are added in both the discriminators and generators to increase training stability and to model long structural dependencies respectively. Network definition, training and inference are performed using the Tensor ow library. All models are trained on a single Nvidia V100 GPU with 32GB of memory and Adam optimization performed for both the generators (lr=1e-4, betal=0.5) and the discriminators (lr=5e-4, beta1=0.5) for 150k iterations. The model is picked based on the best segmentation f1-score on the test set.

### 3.3 Segmentation performance

The inventors first consider the configuration (i) corresponding to a relative shortage of manual annotations.

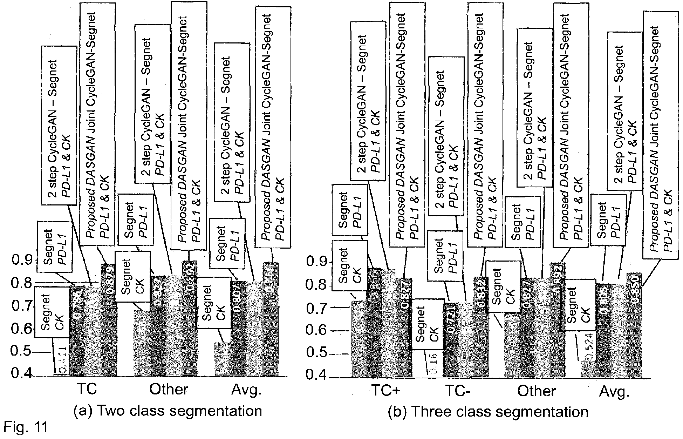

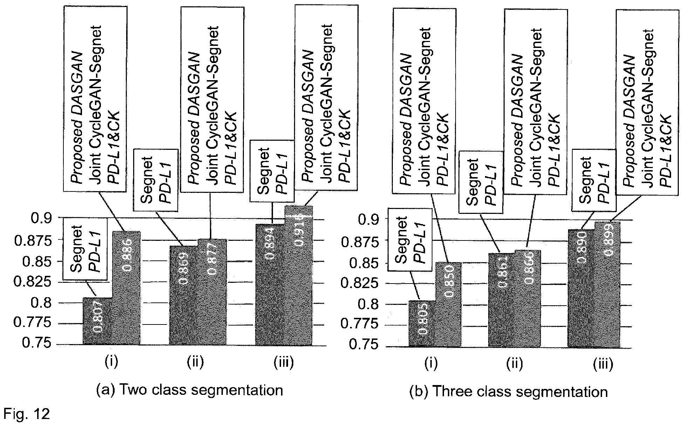

As illustrated in Fig. 11, the proposed end-to-end trainable DASGAN outperforms the two models trained solely on real/synthetic PD-L1 images as well as the model trained on both real and synthetic PD-L1 images using a two step methodology. Mean f1 scores of $f_1$ = 0.886/0.850 are achieved with the proposed DASGAN approach on the binary problem of tumor epithelium detection and on the three class problem of positive and negative tumor epithelial regions respectively. The inventors note that, in their scenario, adding synthetic PD-L1 samples from the CK stained images improves the segmentation accuracy if and only if domain adaptation and semantic segmentation are jointly trained: the two-step methodology does not yield higher accuracy values ($f_1$ = 0.805/0.804) compared to training only on real PD-L1 manual annotations ($f_1$ = 0.807/0.805). As shown in Fig. 12, while the proposed DASGAN model systematically out-performs the baseline model trained only on real PD-L1 samples, the relative improvement in accuracy metrics tends to decrease with the availability of manual annotations. In the configuration (iii) of highest availability, accuracy metrics of $f_1$ =

(0.894/0.890) and $f_1$ = (0.916/0.899) are reached by the baseline model trained only on real PD-L1 samples and by the present approach respectively. This quantitatively confirms the expectation that the use of synthetic data is most relevant in case of relative shortage of manually labeled data.

3.4 Tumor Cell scoring

PD-L1 status, which is predictive for survival of NSCLC patients receiving PD1/PD-L1 checkpoint inhibitor therapy, is determined based on the Tumor Cell score, defined as the percentage of tumor epithelial cells that are PD-L1 positive. The TC score is approximated as the relative area of the detected TC(+) regions:

$$TC_{CNN} = \frac{\#TC(+)}{\#TC(-) + \#TC(+)}. \qquad (6)$$

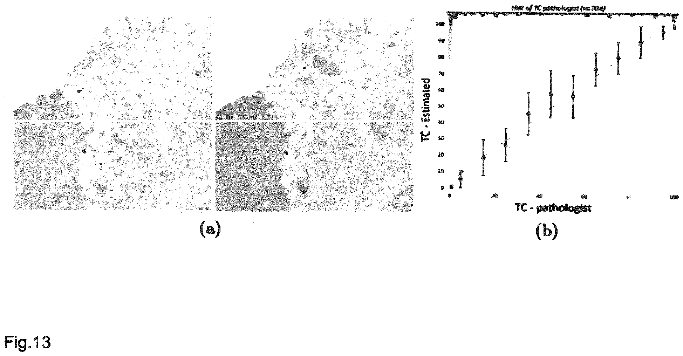

Fig. 13a displays an example of epithelial segmentation output by the proposed DASGAN model. To quantitatively assess the clinical relevance of the proposed approach, the inventors consider a set of 704 PD-L1 stained images unseen for training nor model selection. Fig. 13b shows the bar plot of mean and standard deviation of the estimated TCCNN scores against the true TC scores visually estimated by pathologists. Lin's concordance coefficient of Lcc = 0.93, Pearson correlation coefficient of Pcc = 0.94 and mean absolute error of MAE = 7.30 are reported between the estimated and the true TC score values, showing the high concordance of the proposed method with pathologist scoring.

4 Discussion and Conclusion

The inventors introduce a novel method to leverage data from two stain domains - CK and PD-L1, and use them in conjunction for the semantic segmentation of tumor epithelium in PD-L1 stained images. The possibility to semi-automatically generate large amount of boundary precise annotation in CK stained images, which is enabled by the specificity of this stain to epithelium regions, coupled with the possibility to translate CK stained images into fake but realistic-looking images PD-L1 stained images, which is enabled by the recent advances in the field of unpaired image translation, yields the possible generation of semi-automatically generated dataset for training in the PD-L1 stain domain. As shown experimentally, the proposed DASGAN model, which leverages information from the CK stain domain by jointly solving the two sub-problems of semantic segmentation and domain translation, outperforms previous state-of-the-art approaches and enables both the precise segmentation of tumor epithelium regions and the replication of the clinically relevant PD-L1 Tumor Cell (TC) score. Upon confirmation that the presented results can match survival predictive ability of manual scoring and replication of the findings in a prospective trial, the inventors envision that the proposed method could be used in a clinical setting to identify patients which may benefit from an anti-PD1/PD-L1 therapy. More generally, the inventors believe that the novelty of the proposed DASGAN model for joint domain adaptation and segmentation makes it of interest to the medical image analysis community beyond the analysis of PD-L1 stained histopathology images.

Example 3

Deep Semi Supervised Generative Learning for Automated PD-L1 Tumor Cell Scoring on NSCLC Tissue Needle Biopsies

The level of PD-L1 expression in immunohistochemistry (IHC) assays is a key biomarker for the identification of Non-Small-Cell-Lung-Cancer (NSCLC) patients that may respond to anti PD-1/PD-L1 treatments. The quantification of PD-L1 expression currently includes the visual estimation of a Tumor Cell (TC) score by a pathologist and consists of evaluating the ratio of PD-L1 positive and PD-L1 negative tumor cells. Known challenges like differences in positivity estimation around clinically relevant cut-offs and suboptimal quality of samples makes visual scoring tedious and subjective, yielding a scoring variability between pathologists. In this work, the inventors propose a novel deep learning solution that enables the first automated and objective scoring of PD-L1 expression in late stage NSCLC needle biopsies. To account for the low amount of tissue available in biopsy images and to restrict the amount of manual annotations necessary for training, the inventors explore the use of semi-supervised approaches against standard fully supervised methods. The inventors consolidate the manual annotations used for training as well the visual TC scores used for quantitative evaluation with multiple pathologists. Concordance measures computed on a set of slides unseen during training provide evidence that the present automatic scoring method matches visual scoring on the considered dataset.

1. Introduction

The programmed death receptor 1 (PD-1) checkpoint protein with its ligand - programmed death ligand 1 (PD-L1) plays a major role in the immune escape of the cancerous tumor cells, i.e. in the inhibition of the human immune system responses. More precisely, the proliferation and activation of T-cells as well as the production of the cytokine signaling proteins are inhibited by the binding of PD-L1 proteins to (i) the PD-1 receptors of activated T-cells and (ii) to the CD80/B7-1 receptors on T-cells and antigen presenting cell. Immunotherapeutic drugs aim at restoring the ability of immune cells to kill tumor cells by blocking this escape pathway. The role of complementary or companion diagnostics assays is, in this context, to help the identification of patients which are likely to benefit from a checkpoint inhibitor therapy, i.e. patients with high tumor levels of PD-L1. Tumor level of PD-L1 is estimated by a trained pathologist on biopsies, often obtained with small needles, stained with a PD-L1 antibody. For non-small cell lung carcinoma (NSCLC), which accounts for 85% of all lung cancer related deaths, the Tumor Cell (TC) score is defined as the frequency of PD-L1 expressing tumor cells, i.e. of tumor cells with specifically stained membrane. The tumor level of PD-L1 is then compared to a specific cut-off value, which sets the negative or positive PD-L1 status of the patient.

There are known challenges to an accurate estimation of the Tumor Cell (TC) score. First, PD-L1 staining is not restricted to the membrane of tumor cells: tumor cells with strong cytoplasmic but no membrane staining, immune cells (e.g. macrophage and lymphocyte) as well as necrotic and stromal regions are not included in the score calculation despite possible PD-L1 staining. A challenge specific to visual scoring is moreover the difficulty for any human observer to estimate heterogeneous distribution of cell populations, with positive and negative tumor regions being often spatially inter-mixed. These challenges make TC scoring a subject to some variability among pathologists. In this work, the inventors propose an automatic scoring solution based on image analysis which achieves an accuracy similar to visual scoring while ensuring objective and reproducible scores and could potentially be used as a computer aided system to help pathologists make a better decision. The complexity of the scene and the difficulty of the task naturally lead us to opt for a deep learning-based solution.

Previous works have shown the ability of deep learning-based methods to solve complex tasks in general image analysis and understanding field as well as in the more specific field of digital pathology image analysis. As a first example, it was shown in a pioneering study the potential of deep learning for the detection of prostate cancer regions and of breast cancer metastasis in lymph nodes on digital images of H&E stained slides. More specially, two fully supervised convolutional neural networks (CNN) were independently trained on the complete manual annotations of 200 and 170 tissue slides respectively. Further, a similar fully supervised CNN-based approach for the detection of invasive breast cancer region in H&E stained slides was proposed, relying on the annotations of nearly 400 slides from multiple different sites for training. Most previous works in the field of digital pathology image analysis build on fully supervised networks, which are trained only on labeled information obtained through very extensive manual annotations. Collecting the necessary amount of annotations is however a well-known problem in this field. This is because images with the level of complexity observed in digital pathology can and should only be interpreted by experts with several years of training and experience. This is a key difference to other fields of application of deep learning methods, for instance in robotics, for which the complexity arises more from the number and diversity of the classes than on the ability of untrained humans to recognize these classes. Because pathologists can disagree on the interpretation of a given slide, it is often necessary to collect manual annotations of the same slide from different pathologists. While being often necessary in order to reduce ambiguity in the training set, annotating the same slide multiple times significantly increases the burden of manual annotation.

To bypass the need of manual annotation for region segmentation or object detection, some recent works proposed to directly infer the patient label. A long short term memory (LSTM) network to directly predict patient outcome on tissue microarrays was introduced. A multiple instance learning (MIL) solution to predict prostate cancer diagnosis on needle biopsies was developed, the training of the system requiring a huge dataset of more than 12000 slides. The use of needle biopsies and the small size of datasets available in clinical trial studies make however the use of the aforementioned weakly supervised learning approaches challenging in the present scenario.

As previously shown for the Her2 scoring of breast cancer slides, scores do not have to be learned and can be accurately replicated using the heuristic definition given in the scoring guidelines and an intermediate detection step. To keep the intermediate detection step while reducing the amount of manual annotation, the inventors propose a semi-supervised learning solution. These approaches still employ manual annotations but make use of raw unlabeled data to lower the necessary amount of labeled data. Only a few previous works have used semi-supervised learning for digital pathology image analysis. Recently, a cluster-then-label method based on support vector machine classifier that is shown to outperform classical supervised classifiers was introduced. Further, an image query approach based on semi-supervised manifold learning was proposed. Other works focus on transfer learning where the model weights are initialized on other classification task or learned on raw unlabeled data using representation learning, the labeled data being then used for model refinement only. Given the recent advances in the field of generative adversarial networks (GAN), the inventors' work build on class auxiliary generative adversarial networks (AC-GANs). To the best of the inventors' knowledge, this study introduces the first application of AC-GAN networks for digital pathology image analysis as well as the first computer-aided diagnostic tool for PD-L1 scoring on needle biopsies.

## 2. Methods

The proposed TC scoring algorithm consists of two main steps, i) the detection of positive tumor cell regions (TC(+)) and negative tumor cell regions (TC(-)) using a semi-supervised architecture trained on both labeled and unlabeled data and ii) the computation of the TC score as the ratio between the pixel count of TC(+) regions to the pixel count of the complete tumor cell region. Since area-based approaches often show higher performance than count-based quantification and enable an easier annotation workflow, the inventors estimate the TC scores from the pixel counts of PD-L1 positive and negative tumor regions.

## Dataset consolidation for region detection

A small subset of slides is selected and partially annotated by two pathologists for positive tumor cells TC(+), negative tumor cells TC(-), positive lymphocytes, negative lymphocytes, macrophages, necrosis and stromal regions. A simple detection of the tissue and background regions based on Otsu thresholding32 and morphological filtering is performed, leading to the introduction of another class corresponding to non-tissue regions. Labeled image patches are generated on a regular grid defined on the annotated regions that are concordant between the two pathologists. This leads regions with different classes to be discarded from the analysis. On the remaining set of non-annotated slides, unlabeled patches are generated on a regular grid defined on the detected tissue.

## Auxiliary Classifier Generative Adversarial Networks (AC-GAN)

It is first recalled the principle and architecture of Generative Adversarial Networks. GANs consist of two neural networks, a generator network (G) and a discriminator network (D). The network G transforms a noise vector z, which is sampled from a simple distribution such as an uniform or normal distribution, into an fake image $X_{fake} = G(z)$ using a series of deconvolution and activation layers. The network D classifies an input image as real or fake. More formally, the discriminator outputs the probability distribution over the sources

$$P(S|X), S \in \{real, fake\},$$

through a series of convolutional and activation layers by maximizing the log-likelihood of the correct source:

$$L_S = \mathbb{E}\left[log P(S = real|X_{real})\right] + \mathbb{E}\left[log P(S = fake|X_{fake})\right] \tag{1}$$

The two networks are trained in opposition following a minimax game34 formally defined as follows:

$$\min_G \max_D V(D,G) = \mathbb{E}_{X \sim P_{data}}\left[\log D(X)\right] + \mathbb{E}_{z \sim noise}\left[\log(1 - D(G(z)))\right] \tag{2}$$

More intuitively, the discriminator is trained to differentiate whether the image is coming from real image distribution or fake image distribution from G. In the opposition, G is trained to produce images which are more and more difficult to be identified by the discriminator as real or fake images. When the optimum of this minimax game is reached, the generator creates images so close to the real images that they cannot be differentiated by the discriminator.
The GAN architecture is, however, strictly unsupervised and generative in nature i.e. it can only be used to generate realistic new samples, leading its current main application in digital pathology to be stain normalization. Some recent works in the computer vision community proposed its extension to the AC-GAN variant. The AC-GAN leverages the side information on the class labels by two means. First, the generator is conditioned with class labels to perform conditional image generation:
the generator network takes as input a noise vector concatenated with the one-hot embedded class labels. The concatenated vector goes through a series of transformations by a CNN to create fake images $X_{fake} = G(c; z)$. Second, the discriminator, in addition to predicting the correct source probability P(SIX), also performs class label recovery. The discriminator contains an auxiliary classifier network which outputs the probability distribution of the class P(C|X).
The network parameters between the auxiliary classifier and the discriminator are shared, which enables joint learning of P(CIX) and P(SIX). This requires the introduction of a second cost function as the log-likelihood of the correct

class:

$$L_C = \mathbb{E}\left[logP(C=c|X_{real})\right] + \mathbb{E}\left[logP(C=c|X_{fake})\right]. \qquad (3)$$

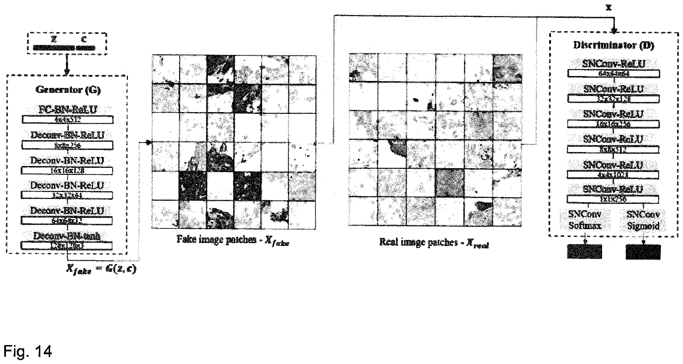

Similar to the vanilla GAN formulation, the two networks D and G are trained by a minimax game with slight modification. The discriminator D is trained to maximize LC + LS and the generator G to minimize LS - LC. Since the maximization and minimization problems are interlaced, the AC-GAN model is trained by alternatively updating the two models G and D. The exact AC-GAN architecture used in this work is displayed in Fig. 14.

In the inventors' application, the inventors are only interested in the classification performance of the network. While the inventors investigate the discriminator performance in more detail, further quantitative assessment of generator has not been performed. Some qualitative examples of images produced by the generator are however displayed in Fig. 14.

Fully-supervised, non-generative and generative semi-supervised networks

The inventors test the semi-supervised generative AC-GAN architecture against two baseline classification networks for fully-supervised learning and two baseline non-generative networks for semi-supervised learning. The two chosen fully-supervised architectures, the inception network v2 and a shallow VGG network modified to be fully-convolutional and to include batch-normalization are commonly employed for the analysis of digital pathology images. The two non-generative semi-supervised networks are vanilla auto-encoder networks built on the two aforementioned classification networks.

3. Experiments and Results

Manual annotation and visual TC scoring datasets

The inventors' dataset consists of 270 NSCLC needle biopsy slides from a subset of the clinical trials (NCT01693562) and (NCT02000947). The slides are stained with the Ventana PD-L1 (SP263) assay and scanned on an Aperio scanner. Scene resolution is 0:198mm per pixel. On a subset of $N_{score}$ = 60 slides, visual TC scoring has been performed on the scanned digital slides by two in-house pathologists. This completes the visual TC scoring on glass-slides obtained from an external source (Ventana Medical System Inc.). A smaller subset Ncnn = 20 is selected across the range of TC scores for training and testing the supervised part of the region detection model and have as such been partially manually annotated by the two in-house pathologists using an in-house annotation software. More specifically, 15 slides are used for training and 5 slides for testing and optimizing the model parameters. The accuracy of the resulting automated TC score is estimated against the three visual TC scores on the remaining $N_{test}$ = 40 unseen slides, i.e. unused for training or testing the supervised nor the unsupervised part of the region detection model.

Model training

Patches of size 128 x 128 pixels are extracted on a regular grid of 20 pixel stride defined on the concordant annotated regions and are augmented using 90 degree rotation. The inventors sample unlabeled patches on a regular grid of 60 pixel stride defined on the tissue area of the 210 slides which have not been scored by all pathologists as well as on the 15 annotated slides which are used for generating the labeled training database. This yields a total of around 180k labeled and 400k unlabeled patches for training as well as 40k labeled patches for testing. All models are trained using the same patches. Batches with 64 labeled patches are used for training the fully-supervised networks. Batches with 32 labeled patches and 32 unlabeled patches are used for training the three semi-supervised networks. For the two non-generative semi-supervised networks, the reconstruction loss is computed on the complete batch and the classification loss on the labeled patches only. For the AC-GAN, the generative loss is computed on the complete batch and the classification loss on the labeled patches only. Training of the four baseline networks and of the generator and discriminator of the AC-GAN network is performed for 100k and 200k iterations respectively using the Adam optimizer with the following learning parameters: lr = 0.0001, beta1 = 0.5, beta2 = C.999. For each network, the inventors select the model weights that maximize the accuracy on the test dataset in order to avoid overfitting on the training set. The developed framework is based on the open-source Keras API and Tensorflow framework.

Prediction and automated TC scoring

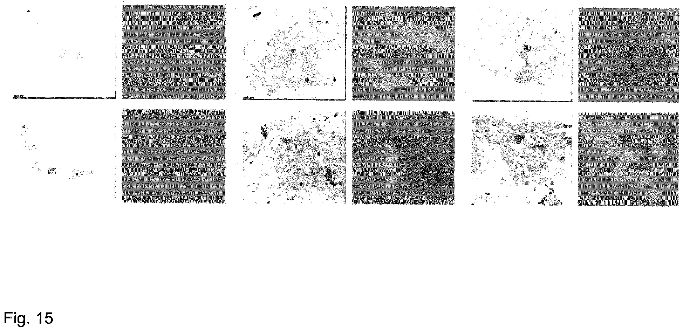

The prediction is restricted to the detected tissue regions and performed in a sliding window manner with a stride of 32 pixels. An example of region detection results is provided in Fig. 15. On each of the $N_{score}$ = 60 slides for which the three visual TC scores are available, the inventors predict the different class probabilities and assign each pixel to the class label of the maximum probability. Given the resulting TC(+) and TC(-) pixels in a given slide, the inventors approximate the corresponding TC score as the ratio of the number of tumor positive cell pixels to the total number of tumor cell pixels:

$$TC_{cnn} = \frac{\#TC(+)}{\#TC(-) + \#TC(+)}. \qquad (4)$$

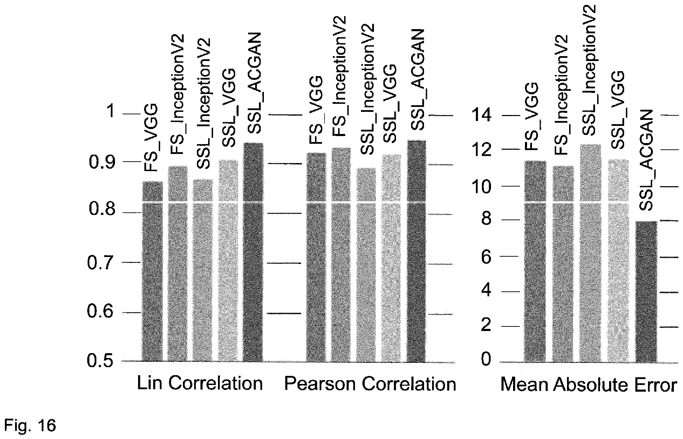

The inventors then compute for each slide the consolidated visual score $TC_{ref.}$ as the median of the three visual scores. The following concordance measures between the automated TC score and the consolidated visual score is calculated on the set of 40 unseen slides only to ensure an independent estimation of the performance: Lin's concordance coefficient (Lcc), Pearson correlation coefficient (Pcc) and Mean Absolute Error (MAE). As reported in Fig. 16, the AC-GAN achieves on all measures a higher level of agreement to the visual scores (Lcc = 0.94, Pcc = 0.95, MSE = 8.03) than any other fully-supervised and semi-supervised network.

Inter-rater variability of visual TC scoring

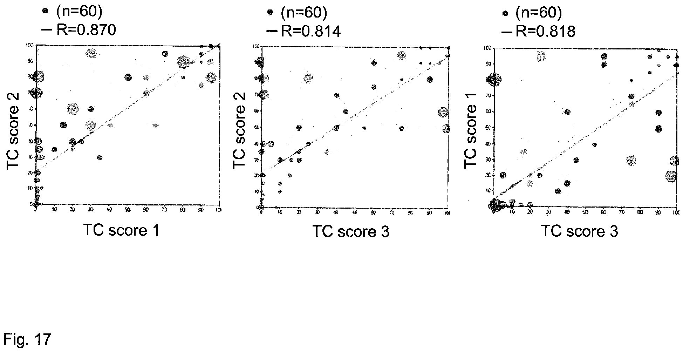

To quantify the variability between the visual scores (cf. Fig. 17), the inventors additionally estimate for each slide the inter-rater variability $\Delta_{path.}$ as the mean absolute pairwise difference between the associated visual scores:

$$\Delta_{path.} = \frac{1}{6} \sum_{\substack{1 \le i < 3 \\ i < j \le 3}} |TC_{path_i} - TC_{path_j}|. \qquad (5)$$

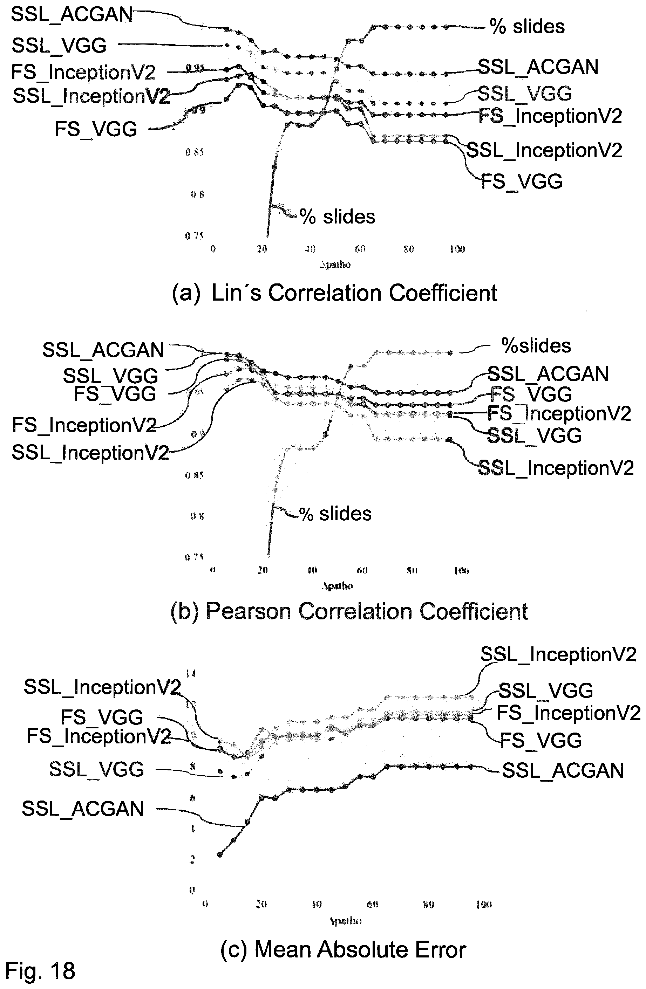

The very high concordance of PD-L1 scoring on glass slides and digital slides has recently been confirmed, leading the inventors to pool of all data to estimate inter-rater variability independently of a glass/digital scoring. Given increasing maximum levels of inter-rater variability, the inventors restrict the computation of the aforementioned concordance measures to the slides whose associated value stays below the given maximum. As displayed in Fig. 18, the automated TC scores become more concordant to the consolidated visual scores the more concordant the visual scores are. A Lin's concordance coefficient of 0.96 is for example reached on the TC scores obtained with the AC-GAN architecture on cases for which the inter-rater variability is smaller than 40%. The better performance of the semi-supervised generative AC-GAN network over the other networks is consistent across highly-concordant and low-concordant cases.

Automated AC-GAN scoring and visual TC scoring

The inventors compare hereby in more detail the performance of the automated score based on the AC-GAN detection to the three visual scores by pathologists. Table 1 reports the same concordance measures as above between all the visual scores and the AC-GAN score. To study the ability of the proposed automated solution to determine the patient status, the inventors additionally compute the Overall Percent Agreement (OPA), Negative Percent Agreement (NPA) and Positive Percent Agreement (PPA) at the 25% cut-off (cf. Table 2). This cut-off value has been shown to optimize the probability of responses to durvalumab in NSCLC.

The TC score computed from the AC-GAN predicted regions yields for all but one case to the highest correlation and the lowest absolute error. Note that in the only case where this does not hold, the Pearson correlation of the automated score $TC_{cnn}$ = 89 is very close to the highest value $TC_1$ = 0.90. Computing the concordance to the visual scores $TC_3$ estimated on the microscope, the inventors note that the automated TC scores leads to a higher agreement (Lcc=0.88, Pcc=0.89, MAE=11.3) than the two pathologist scores estimated on digital slides ($TC_1$:Lcc=0.81, Pcc=0.82, MAE=13.2) - ($TC_2$:Lcc=0.79, Pcc=0.82, MAE=16.6). These observations are confirmed on the reported OPA values of the low/high PD-L1 status at 25% cut-off. The automated scoring systematically yields the highest agreement with the visual TC score. In particular, considering the third visual TC scoring, automated scoring achieves an OPA of 0.88 to be compared with OPAs of 0.88 and 0.80 observed for the first and second pathologists visually scoring on digital slides.

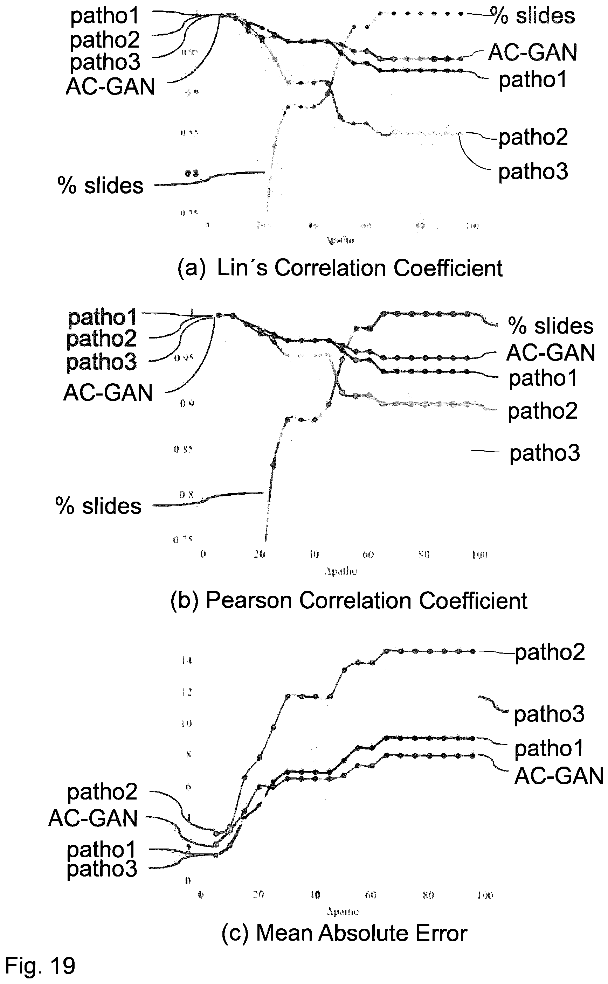

To further analyze the concordance of automated scoring versus visual scoring, the inventors consider the AC-GAN score and the three visual scores independently of their source and compute for each of the four resulting scores all concordance measures between each score and the median of the three remaining scores. Results displayed in Table 3 and Fig. 19 provide further evidence of the good performance of the automated TC score. In all measures, the automated TC scores systematically outperform the visual scores.

| | $TC_1$ | | | $TC_2$ | | | $TC_3$ | | | $TC_{cnn}$ | | |
|---|---|---|---|---|---|---|---|---|---|---|---|---|
| | Lcc | Pcc | MAE | Lcc | Pcc | MAE | Lcc | Pcc | MAE | Lcc | Pcc | MAE |
| $TC_1$ | - | - | - | 0.84* | 0.90* | 15.3 | 0.81 | 0.82 | 13.2 | 0.95* | 0.95* | 7.4* |
| $TC_2$ | 0.84 | 0.90 | 15.3 | - | - | - | 0.79 | 0.82 | 16.6 | 0.84 | 0.89 | 15.0 |
| $TC_3$ | 0.81 | 0.82 | 13.2 | 0.79 | 0.82 | 16.6 | - | - | - | 0.88 | 0.89 | 11.3 |
| $TC_{cnn}$ | 0.95* | 0.95* | 7.4* | 0.84* | 0.89 | 15.0* | 0.88* | 0.89* | 11.3* | - | - | - |

Table 1. Pairwise Lin's concordance / Pearson correlation coefficients and Mean Absolute Error (Lcc/Pcc/MAE) between all visual TC scores and estimated TC score based on AC-GAN. The star indicates for each concordance measure the TC score yielding the best performance, i.e. the maximum value for Lcc and Pcc and the minimum value for the MAE. Values are computed on the $N_{test}$ cases unseen during training or testing of the AC-GAN model.

| | $TC_1$ | | | $TC_2$ | | | $TC_3$ | | | $TC_{cnn}$ | | |
|---|---|---|---|---|---|---|---|---|---|---|---|---|
| | OPA | NPA | PPA | OPA | NPA | PPA | OPA | NPA | PPA | OPA | NPA | PPA |
| $TC_1$ | - | - | - | 0.78 | 0.62 | 1.00 | 0.88* | 0.88 | 0.88* | 0.90* | 0.88 | 0.94* |
| $TC_2$ | 0.78 | 1.00* | 0.65 | - | - | - | 0.80 | 1.00* | 0.69 | 0.83 | 1.00* | 0.73 |
| $TC_3$ | 0.88 | 0.91 | 0.83 | 0.80 | 0.65 | 1.00 | - | - | - | 0.88 | 0.87 | 0.89 |
| $TC_{cnn}$ | 0.90* | 0.95 | 0.84* | 0.83* | 0.68* | 1.00* | 0.88* | 0.91 | 0.84 | - | - | - |

Table 2. Pairwise Overall, Negative and Positive Percent Agreement (OPA/NPA/PPA) at the 25% cut-off between all visual TC scores and estimated TC score based on AC-GAN. The star indicates for each concordance measure the TC score yielding the best performance, i.e. the maximum value.

Values are computed on the $N_{test}$ cases unseen during training or testing of the AC-GAN model.

| - | $TC_1$ | $TC_2$ | $TC_3$ | $TC_{cnn}$ |
|---|---|---|---|---|
| Lin / Pearson / MAE | 0.83 / 0.94 / 9.14 | 0.85 / 0.90 / 14.65 | 0.85 / 0.85 / 11.73 | 0.94 / 0.95 / 8.00 |
| OPA / NPA / PPA | 0.85 / 0.95 / 0.76 | 0.78 / 0.63 / 1.0 | 0.85 / 0.90 / 0.80 | 0.88 / 0.90 / 0.85 |

Table 3. Concordance measures between the visual and automated TC scores against the median of the other scores on the $N_{test}$ cases unseen during training or testing of the AC-GAN model.

## 4. Discussion and Conclusion

The aim of anti-PDL1 therapies is to revive the immune response to cancer cells: inhibiting the PD-L1 pathway reverses T-cell exhaustion and restores T cell's cytotoxic activity. Patients with high expression generally showing higher response rates and longer progression free survival than patients with low expression, an accurate testing of PD-L1 expression may help take the best treatment decision on whether or not to follow such therapy. There is a significant heterogeneity between the existing PD-L1 expression tests, different antibodies being employed as companion and complementary diagnostics for different immunotherapeutic drugs, different patterns of staining (tumor cells only or tumor cells and tumor infiltrating immune cells) being considered for different indications (NSCL resp. urothelial carcinoma) and finally different cutoffs being used for different antibodies for a given indication.

While the focus of this work is only to replicate the test corresponding to the antibody clone SP263 on NSCLC patients, the inventors present what is to the inventors' knowledge the first proof of concept study showing that deep learning enables an accurate and automated estimation of the PD-L1 expression level and PD-L1 status on biopsies samples. The performed analysis of inter-rater variability suggests that the accuracy achieved by the proposed automated scoring method is concordant with visual scoring by pathologists on the present dataset. This work focuses on the automated estimation of the PD-L1 TC score yet, it more generally introduces the first application of deep semi-supervised and generative learning networks (AC-GAN) in the field of digital pathology. It also provides first evidence of the good performance of this model against standard fully supervised learning networks.

Going beyond the presented proof of concept, the inventors believe that further evidence could be provided by increasing the size of the unseen dataset on which the agreement of the automated and visual TC scores has been computed as well as ensuring that the comparison between the visual scores is not biased by external parameters such as (i) the heterogeneous experience of the pathologists and (ii) if the scoring is performed on digital or glass slides. The presented work also opens the way for PD-L1 scoring in bladder cancer which involves the scoring of both tumor cells and immune cell regions and for which the reliability among pathologists is extremely weak8. In general, the inventors envision that the success of checkpoint inhibitor related immune therapies can be increased by automated profiling of tumor and immune cells with respect to their cluster of differentiation (CD) protein expression levels. This study on CD274 (PD-L1) in NSCLC is the first conclusive step in this journey.

In the following, further information relating to various aspects of Example 3 is provided.

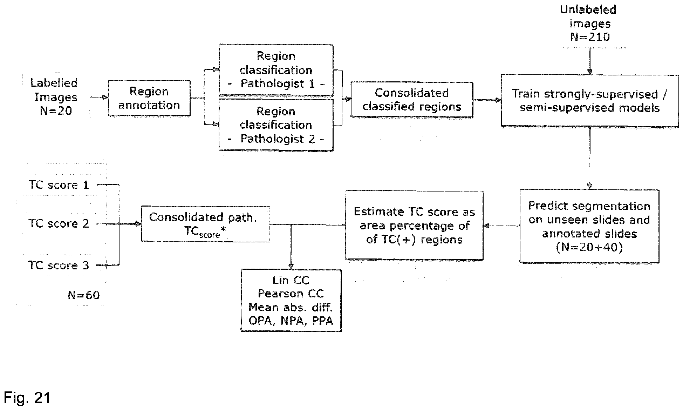

FIG. 21 shows an overview of the method of generating a Tumor Cell (TC) score of a cancer patient.

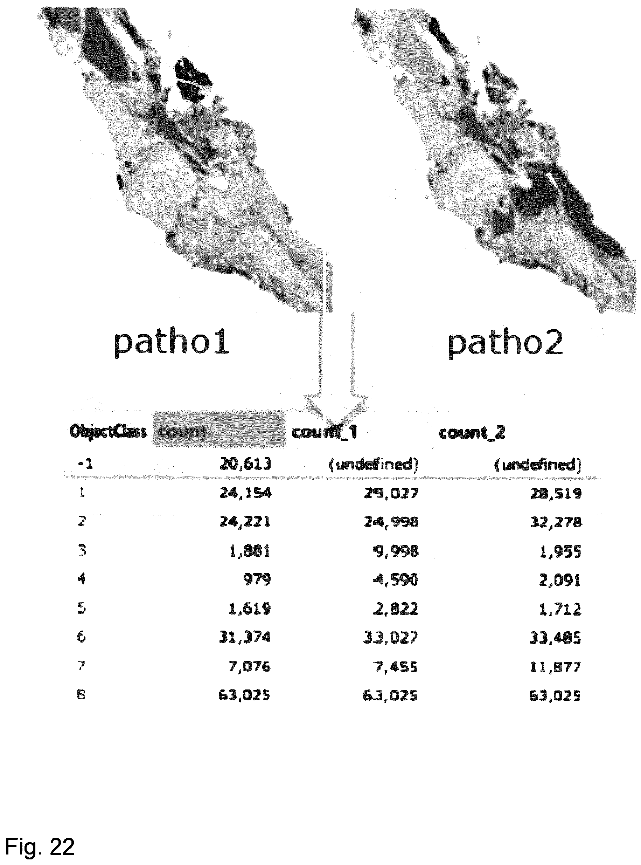

FIG. 22 shows the variability of region annotation of tumor tissue biopsy slides between two pathologists (pathol, patho2). Partial region annotation (class assignment) of 20 biopsy slides was performed by two pathologists. Seven classes of objects were annotated: positive tumor cells (TC(+)), negative tumor cells (TC(-)), positive lymphocytes (Lymph(+)), negative lymphocytes (Lymph(-)), macrophages (Mac(+)), stroma and necrosis. Additional automatic detection of background class was performed. It was found that inter-pathologist variability with a very high variability (about 20% disagreement) exists. To reduce noise, only concordant annotations are kept. The Veritrova setup and patch generation workflows were adapted.

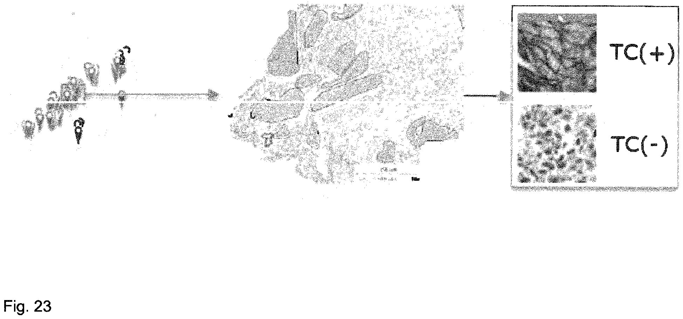

FIG. 23 relates to Strongly Supervised Learning. Only annotated images are considered. Success depends on the availability of a large amount of manual annotations. TC, tumor cell.

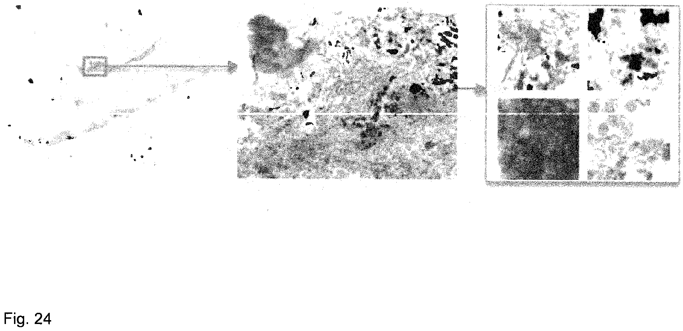

FIG. 24 relates to Semi-Supervised Learning. In contrast to Strongly Supervised Learning, only a few images are annotated. The use of unlabeled images improves the learning of visual features.

Semi-Supervised Learning involves classification networks, autoencoders and semi-supervised autoencoders.

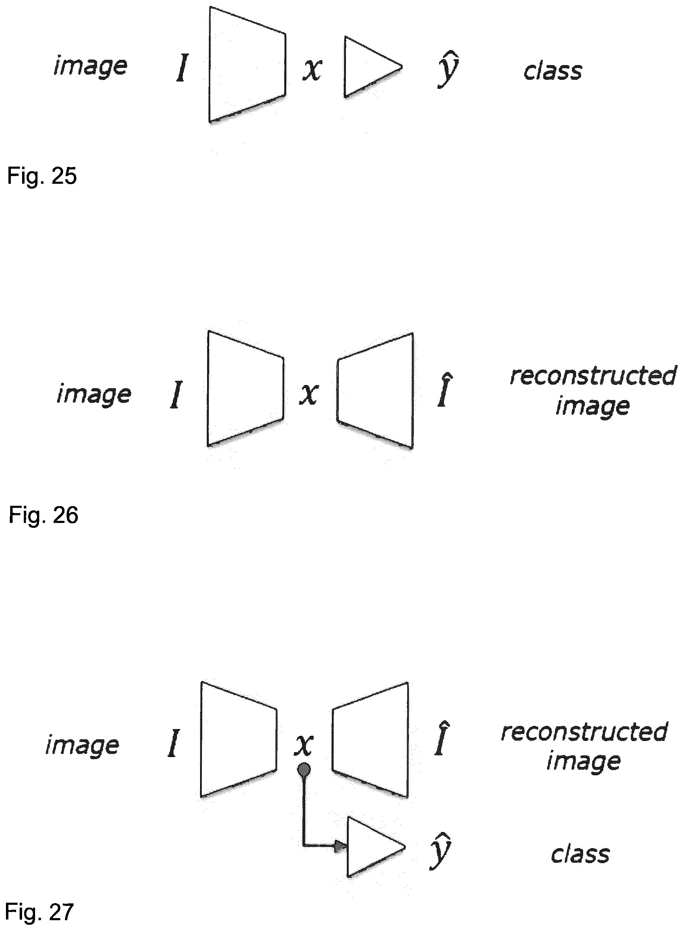

FIG. 25 relates to classification networks with compression of the input image into a low dimensional representation (feature space), classification network from the low dimensional reduction to the class label, and minimizing the classification error.

FIG. 26 relates to autoencoders with compression of the input image into a low dimensional representation, decompression back into a "reconstructed input", and minimizing the reconstruction error between the original and the reconstructed inputs.

FIG. 27 relates to semi-supervised autoencoders with minimizing both the reconstruction error and the classification error.

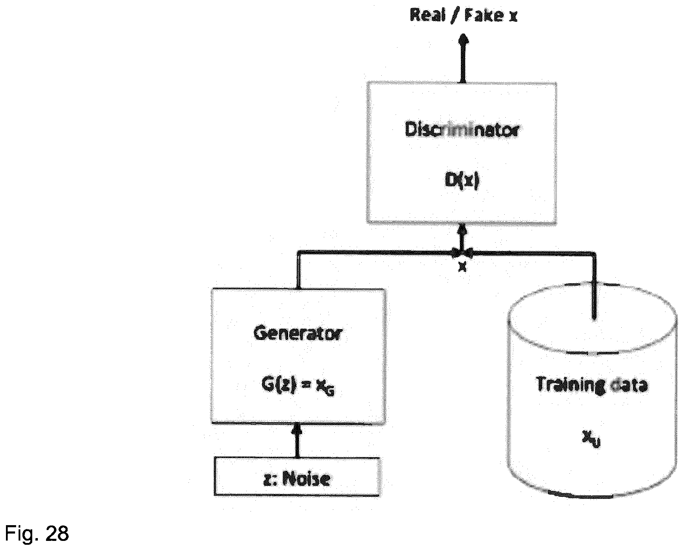

FIG. 28 relates to Generative Adversarial Networks (GANs). Artificial but real-looking images are generated. The Generator serves to "forge" artificial images from noise. The Discriminator serves to "find out" if True or Fake image.

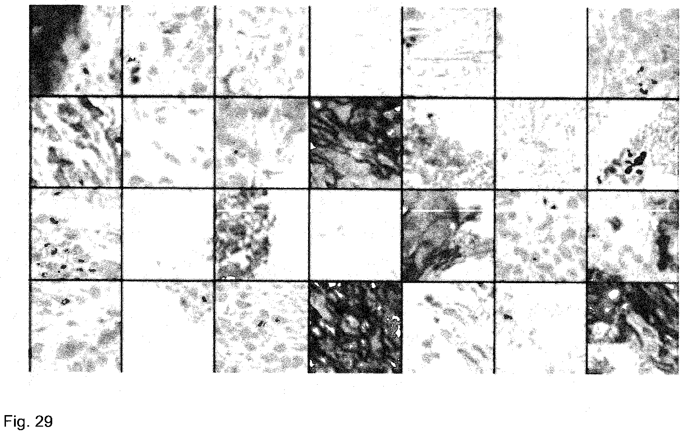

FIG. 29 shows generated artificial but real-looking PD-L1 images.

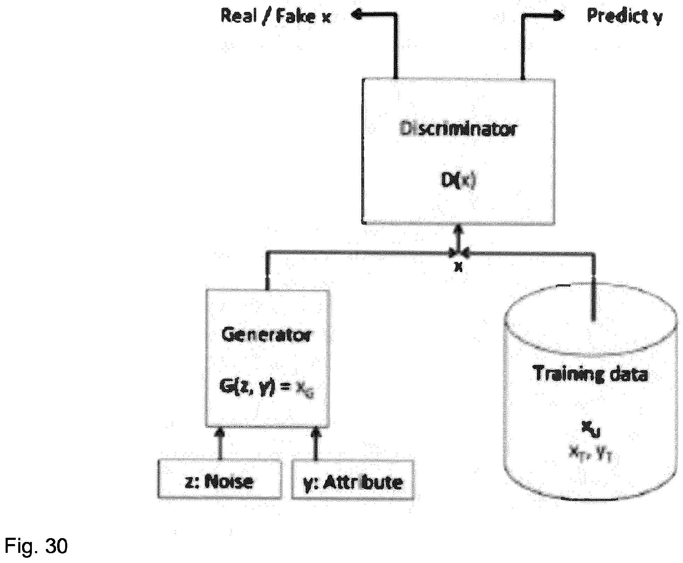

FIG. 30 relates to Auxiliary Classifier GANs (AC-GANs). Real-looking images are generated and class is predicted. The Generator serves to "forge" artificial images from noise. The Discriminator serves to "find out" if True or Fake image and to predict the class label.

Regarding region segmentation, fully convolutional networks for semantic segmentation are described in Long et al. 2014, learning deconvolution networks for semantic segmentation are described in Noh et al. 2015, and the use of deep learning for segmentation and counting within microscopy data is described in Hernández et al. 2018.

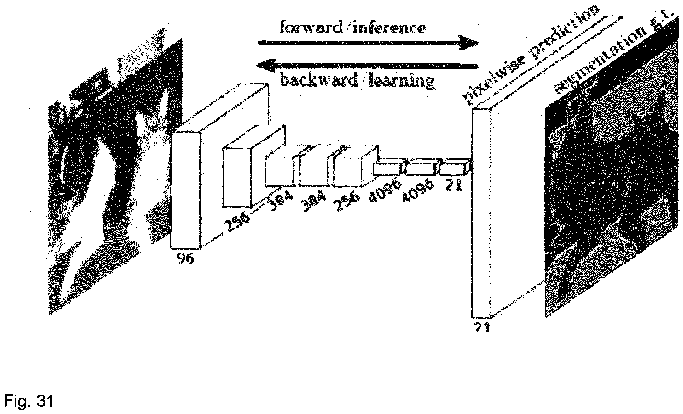

FIG. 31 relates to Semantic Segmentation Networks. It is taken from Long et al. 2014. The network can be adapted for dense prediction by replacing the fully connected layers by convolutional layers. The resulting network outputs a coarse classification heatmap, i.e. provides class prediction not only for the center pixel of the image patch but a subset of equally spaced pixels in the image patch. Applying the network multiple times and aggregating the resulting coarse heatmap in a "shift and stich" fashion or applying a deconvolution enable the generation of dense classification heatmaps.

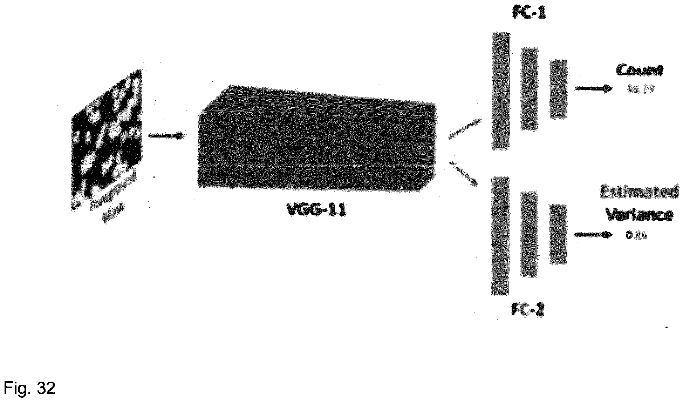

FIG. 32 relates to Regression networks. It is taken from Hernández et al. 2018. The network can be adapted for regression instead of classification by replacing the classification layer e.g. by a regression layer at the end of the network. While the classification network enables the identification of the class of the center pixel (resp. of all individual pixels) in the image patch, the regression network is used to directly estimate the number of pixels or the number of cells in the image patch.

Model Training involves patches with class label, patches without class label and comparing five different networks. Patches with class label are based on 20 partially annotated biopsy images (15 for train, 5 for test), only on samples for which both pathologists agree on the class. There are 8 classes, currently unbalanced. This leads to 187k training patches and 42k testing patches for supervised learning. Patches without class label are based on 210 slides without manual annotations nor TC score by all the three pathologists, the 15 slides with class label which are also used for training the supervised training networks. This leads to 390k patches from unsupervised learning. Comparing five different networks involves Strongly Supervised (LeNet, InceptionV2) and Semi-Supervised (AE-LeNet, AE-InceptionV2, AC-GAN).

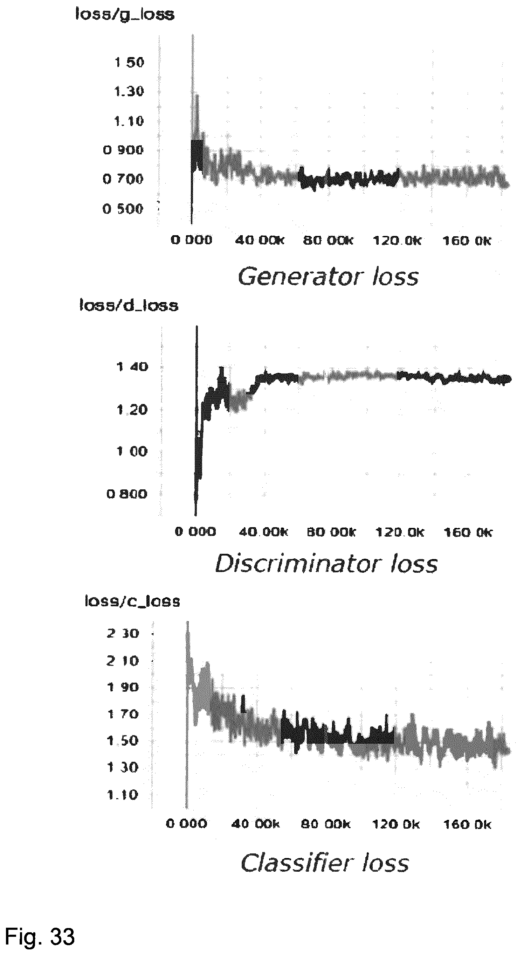

FIG. 33 shows Generator loss, Discriminator loss and Classifier loss.

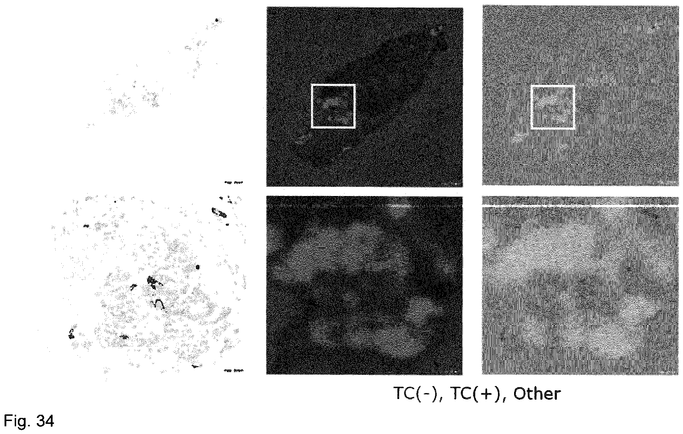

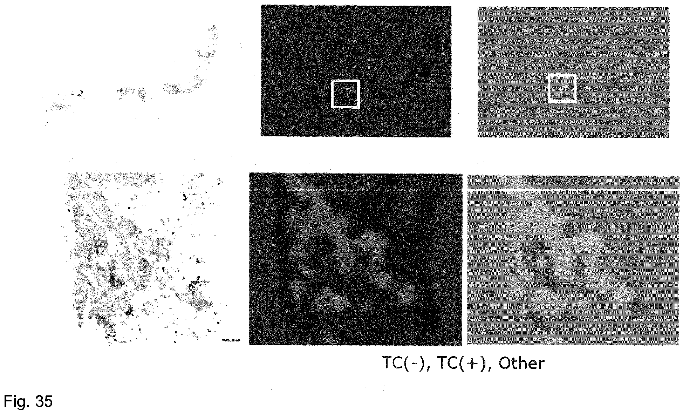

FIG. 34 and FIG. 35 show examples of region segmentation.

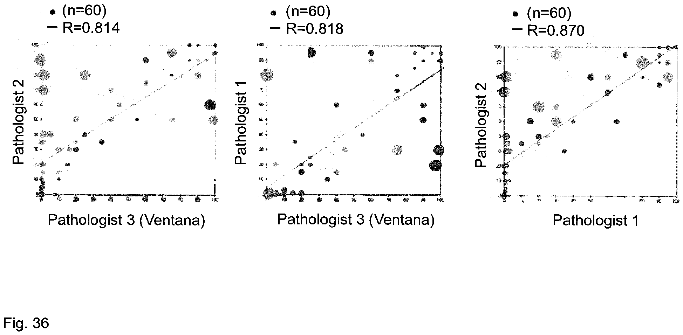

FIG. 36 relates to inter-pathologist variability. In case of a TC score by a single pathologist, the inter-pathologist variability is unknown. In case of TC scores by three pathologists, there is an additional scoring by two pathologists. This enable the estimation of inter-pathologist variability. The inventors analyzed whether TC scores based on image analysis can be distinguished from TC scores by a human pathologist. To estimate inter-pathologist variability, the mean divergence between TC scores by two pathologists was computed as follows:

$$\Delta patho = \sum_{i \neq j} (TC_{path\_i} - TC_{path\_j})$$

For a consolidated pathologist, the mean of the TC scores by the three human pathologists is computed and the consolidated TC score is set as the TC score which is the closest to this mean.

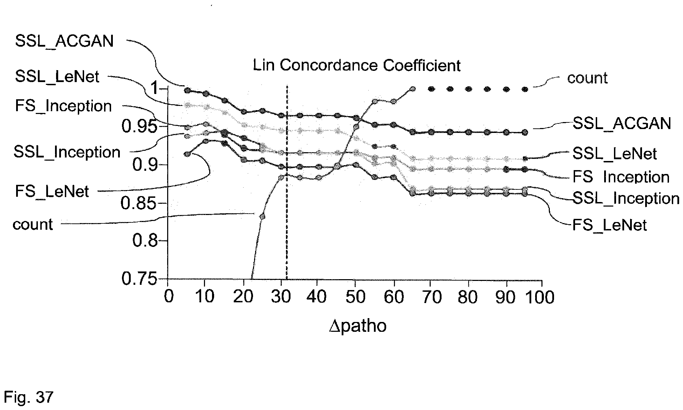

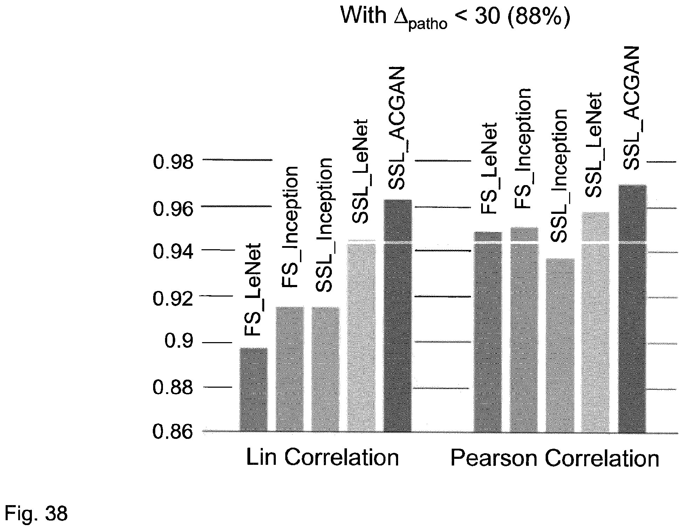

For choosing the best model, Lin and Pearson correlations for increasing value of allowed inter-pathologist variabilty ($\Delta patho$) was computed. The results are shown in FIG. 37 and FIG. 38. It was found that the semi-supervised model AC-GAN systematically yields the best accuracy.

The semi-supervised model AC-GAN was compared with a pathologist. To this end, the AC-GAN model is considered to be a fourth pathologist. To see whether it can be distinguished from the three human pathologists, for each of the four (3+1) pathologists:

(1) the TC scores by the current pathologist is discarded,
(2) for each patient, the mean of the TC scores by the three remaining pathologists is computed,
(3) the consolidated TC score is set as the TC score which is the closest to this mean, and

(4) OPA, NPA, PPA, LinCC, between the current TC scores and the consolidated TC scores are computed.

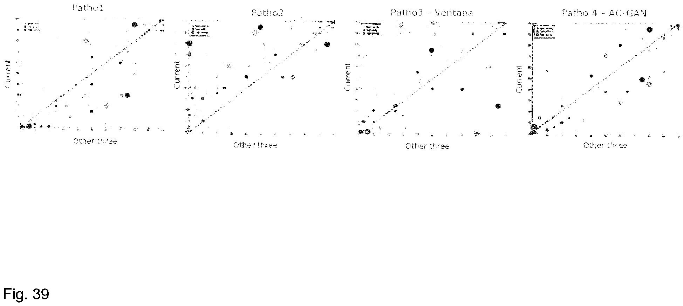

The results are shown in FIG. 39.

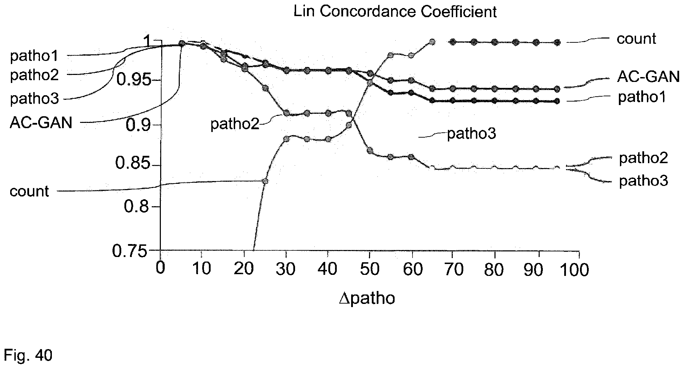

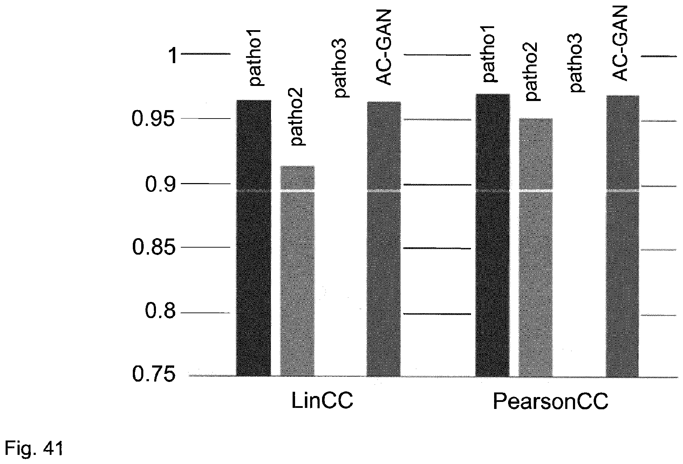

Lin concordance (LinCC) and Pearson correlation (PearsonCC) are computed. The results are shown in FIG. 40 and FIG. 41.

It was found that the AC-GAN model is as good as a human pathologist with respect to Lin concordance, Pearson correlation, and mean absolute difference.

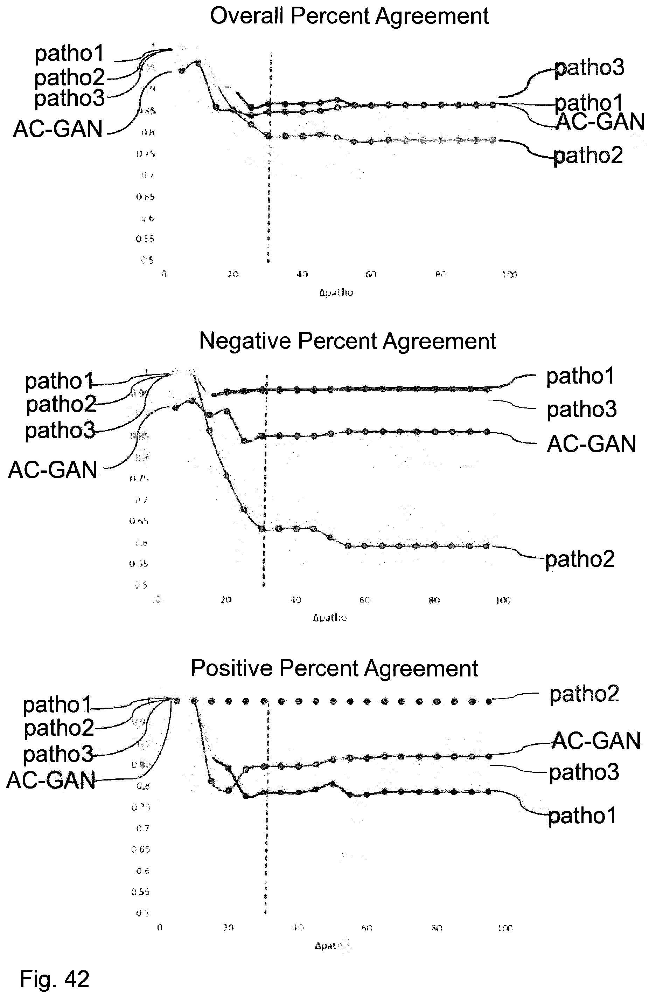

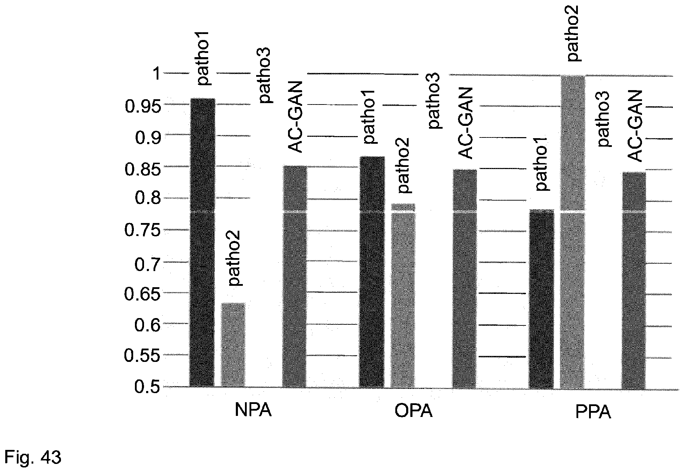

Overall Percent Agreement (OPA), Negative Percent Agreement (NPA), and Positive Percent Agreement (PPA) are computed at 25% cut-off. The results are shown in FIG. 42 and FIG. 43. It was found that the AC-GAN model is as good as a human pathologist with respect to OPA, NPA, PPA at 25% cut-off.

REFERENCES

Hernández, C. X., Sultan, M. M., and Pande, V. S. Using deep learning for segmentation and counting within microscopy data. arXiv preprint arXiv: 1802.10548, 2018.

Long, J., Shelhamer, E., and Darrel, T. Fully convolutional networks for semantic segmentation. CoRR, abs/1411.4038, 2014.

Noh, H., Hong, S., and Han, B. Learning deconvolution network for semantic segmentation. In: ICCV. pp. 1520-1528, 2015.

**Claims**

1. A method of generating a score of a histopathological diagnosis of a cancer patient, comprising:

    loading a first image patch into a processing unit, wherein the first image patch is cropped from a digital image of a slice of tissue, and wherein the slice of tissue has been immunohistochemically stained using a diagnostic antibody;
    determining how many pixels of the first image patch belong to a first tissue that has been positively stained by the diagnostic antibody, how many pixels of the first image patch belong to a second tissue that has been negatively stained by the diagnostic antibody, and how many pixels of the first image patch belong to a third tissue that is neither the first tissue nor the second tissue, wherein the determining is performed by processing the first image patch using a convolutional neural network;
    processing, using said convolutional neural network, additional image patches that have been cropped from the digital image to determine how many pixels of each additional image patch belong to the first tissue, how many pixels of each additional image patch belong to the second tissue, and how many pixels of each additional image patch belong to the third tissue;
    computing the score of the histopathological diagnosis based on a total number of pixels that belong to the first tissue; and
    displaying the digital image and the score on a graphical user interface, wherein the convolutional neural network is trained using generative adversarial learning,
    wherein the convolutional neural network is trained using two generative adversarial networks forming a cycle, wherein a first of the two generative adversarial networks transforms image patches generated on a stain domain A into fake patches of a stain domain B, and wherein a second of the two generative adversarial networks transforms image patches generated on the stain domain B into fake patches of the stain domain A,
    wherein the first of the two generative adversarial networks performs segmentation on the stain domain B after the transformation of the image patches generated on the stain domain A into fake patches of the stain domain B, and wherein the second of the two generative adversarial networks performs segmentation on the stain domain A after the transformation of the image patches generated on the stain domain B into fake patches of the stain domain A
    wherein the score is a ratio of the total number of pixels that belong to the first tissue to a total number of pixels that belong to either the first tissue or the second tissue, wherein the stain domain A is the tissue or the region of a digital image of a tissue that stains for a specific biomarker or stain and wherein stain domain B is the tissue or the region of the digital image of the tissue that stains for the diagnostic antibody, and
    wherein the first and the second tissue both stain for the specific biomarker or stain.

2. The method of claim 1, wherein the slice of tissue is taken from a biopsy of lung cancer.

3. The method of either claim 1 or claim 2, wherein the diagnostic antibody is taken from the group consisting of: HER2, PD-L1, PD-L2, and CD73.

4. The method of any one of claims 1 to 3, wherein the diagnostic antibody targets a protein, further comprising:

   selecting a therapy if the score obtained using the diagnostic antibody is larger than a predetermined threshold, wherein the therapy that is selected uses a therapeutic antibody that binds to the protein targeted by the diagnostic antibody.

5. The method of claim 4, wherein the diagnostic antibody is PD-L1, and wherein the selected therapy uses a PD-L1 medication if the score is larger than the predetermined threshold.

6. The method of any one of claims 1 to 5, wherein the first tissue that has been positively stained by the diagnostic antibody includes cells taken from the group consisting of: epithelial cells, endothelial cells, stromal cells, macrophages, T cells and B cells.

7. The method of any one of claims 1 to 6, wherein the stain domain A is the tissue or the region of a digital image of a tissue that stains for a cell-type specific marker.

8. The method of any one of claims 1 to 7, wherein the convolutional network acts as a discriminator in the cycle formed by the two generative adversarial networks.

9. A system that generates a score of a histopathological diagnosis for a cancer patient, comprising: code for loading a first image patch into a processing unit, wherein the first image patch is cropped from a digital image of a slice of tissue, and wherein the slice of tissue has been immunohistochemically stained using a diagnostic antibody; code for determining how many pixels of the first image patch belong to a first tissue that has been positively stained by the diagnostic antibody, how many pixels of the first image patch belong to a second tissue that has been negatively stained by the diagnostic antibody, and how many pixels of the first image patch belong to a third tissue that is neither the first tissue nor the second tissue, wherein the determining is performed by processing the first image patch using a convolutional neural network; code for processing, using said convolutional neural network, additional image patches that have been cropped from the digital image to determine how many pixels of each additional image patch belong to the first tissue, how many pixels of each additional image patch belong to the second tissue, and how many pixels of each additional image patch belong to the third tissue; code for computing the score of the histopathological diagnosis based on a total number of pixels that belong to the first tissue; and a graphical user interface that displays the digital image and the score, wherein the convolutional neural network is trained using generative adversarial learning, wherein the convolutional neural network is trained using two generative adversarial networks forming a cycle, wherein a first of the two generative adversarial networks transforms image patches generated on a stain domain A into fake patches of a stain domain B, and wherein a second of the two generative adversarial networks transforms image patches generated on the stain domain B into fake patches of the stain domain A, wherein the first of the two generative adversarial networks performs segmentation on the stain domain B after the transformation of the image patches generated on the stain domain A into fake patches of the stain domain B, and wherein the second of the two generative adversarial networks performs segmentation on the stain domain A after the transformation of the image patches generated on the stain domain B into fake patches of the stain domain A, wherein the score is a ratio of the total number of pixels that belong to the first tissue to a total number of pixels that belong to either the first tissue or the second tissue, wherein the stain domain A is the tissue or the region of a digital image of a tissue that stains for a specific biomarker or stain and wherein stain domain B is the tissue or the region of the digital image of the tissue that stains for the diagnostic antibody, and wherein the first and the second tissue both stain for the specific biomarker or stain.

10. The system of claim 9, wherein if the score is larger than a predetermined threshold, then the system recommends a therapy that uses a therapeutic antibody that targets the same protein as that targeted by the diagnostic antibody.

11. The system of claim 10, wherein the diagnostic antibody is PD-L1, and wherein the therapy uses a drug targeting a protein of the group consisting of PD-L1, PD-1.

**Patentansprüche**

1. Verfahren zum Generieren einer Auswertung für die histopathologische Diagnose eines Krebspatienten, umfassend:

   Laden eines ersten Bildausschnitts in eine Verarbeitungseinheit, wobei der erste Bildausschnitt aus einem digitalen Bild einer Gewebescheibe geschnitten wird und wobei die Gewebescheibe immunhistochemisch unter Verwendung eines diagnostischen Antikörper angefärbt wurde;
   Bestimmen, wie viele Pixel des ersten Bildausschnitts zu einem ersten Gewebe gehören, das durch den diagnostischen Antikörper positiv angefärbt wurde, wie viele Pixel des ersten Bildausschnitts zu einem zweiten Gewebe gehören, das durch den diagnostischen Antikörper negativ angefärbt wurde, und wie viele Pixel des ersten Bildausschnitts zu einem dritten Gewebe gehören, das weder das erste Gewebe noch das zweite Gewebe ist, wobei das Bestimmen durch Verarbeiten des ersten Bildausschnitts unter Verwendung eines neuronalen Faltungsnetzwerks durchgeführt wird;
   Verarbeiten, unter Verwendung des neuronalen Faltungsnetzwerks, zusätzlicher Bildausschnitte, die aus dem digitalen Bild geschnitten wurden, um zu bestimmen, wie viele Pixel jedes zusätzlichen Bildausschnitts zu dem ersten Gewebe gehören, wie viele Pixel jedes zusätzlichen Bildausschnitts zu dem zweiten Gewebe gehören und wie viele Pixel jedes zusätzlichen Bildausschnitts zu dem dritten Gewebe gehören;
   Berechnen der Auswertung der histopathologischen Diagnose basierend auf einer Gesamtzahl von Pixeln, die zu dem ersten Gewebe gehören; und
   Anzeigen des digitalen Bilds und der Auswertung auf einer grafischen Benutzeroberfläche,
   wobei das neuronale Faltungsnetzwerk unter Verwendung von generativ-adversativem Lernen trainiert wird,
   wobei das neuronale Faltungsnetzwerk unter Verwendung zweier generativ-adversativer Netzwerke trainiert wird, die einen Zyklus ausbilden, wobei ein erstes der zwei generativ-adversativen Netzwerke Bildausschnitte, die auf einer Farbstoffdomäne A generiert werden, in gefälschte Ausschnitte einer Farbstoffdomäne B transformiert, und wobei ein zweites der zwei generativ-adversativen Netzwerke Bildausschnitte, die auf der Farbstoffdomäne B generiert werden, in gefälschte Ausschnitte der Farbstoffdomäne A transformiert,
   wobei das erste der zwei generativ-adversativen Netzwerke eine Segmentierung auf der Farbstoffdomäne B nach der Transformation der Bildausschnitte, die auf der Farbstoffdomäne A generiert werden, in gefälschte Ausschnitte der Farbstoffdomäne B durchführt, und wobei das zweite der zwei generativ-adversativen Netzwerke die Segmentierung auf der Farbstoffdomäne A nach der Transformation der Bildausschnitte, die auf der Farbstoffdomäne B generiert werden, in gefälschte Ausschnitte der Farbstoffdomäne A durchführt,
   wobei die Auswertung ein Verhältnis der Gesamtzahl von Pixeln, die zu dem ersten Gewebe gehören, zu einer Gesamtzahl der Pixel ist, die entweder zu dem ersten Gewebe oder dem zweiten Gewebe gehören, wobei die Farbstoffdomäne A das Gewebe oder der Bereich eines digitalen Bilds eines Gewebes ist, der für einen spezifischen Biomarker oder Farbstoff angefärbt wird, und wobei die Farbstoffdomäne B das Gewebe oder der Bereich des digitalen Bilds des Gewebes ist, der für den diagnostischen Antikörper angefärbt wird, und wobei sowohl das erste als auch das zweite Gewebe den spezifischen Biomarker oder Farbstoff aufweisen.

2. Verfahren nach Anspruch 1, wobei die Gewebescheibe aus einer Biopsie von Lungenkrebs genommen wird.

3. Verfahren nach Anspruch 1 oder 2, wobei der diagnostische Antikörper aus der Gruppe genommen wird, bestehend aus: HER2, PD-L1, PD-L2 und CD73.

4. Verfahren nach einem der Ansprüche 1 bis 3, wobei der diagnostische Antikörper auf ein Protein abzielt, ferner umfassend:
   Auswählen einer Therapie, falls die Auswertung, die unter Verwendung des diagnostischen Antikörpers erhalten wird, größer als ein zuvor bestimmter Schwellenwert ist, wobei die Therapie, die ausgewählt wird, einen therapeutischen Antikörper verwendet, der an das Protein bindet, auf das durch den diagnostischen Antikörper abgezielt wird.

5. Verfahren nach Anspruch 4, wobei der diagnostische Antikörper ein PD-L1 ist und wobei die ausgewählte Therapie ein PD-L1-Medikament verwendet, falls die Auswertung größer als der zuvor bestimmte Schwellenwert ist.

6. Verfahren nach einem der Ansprüche 1 bis 5, wobei das erste Gewebe, das durch den diagnostischen Antikörper positiv angefärbt wurde, Zellen einschließt, die aus der Gruppe genommen werden, bestehend aus: Epithelzellen, Endothelzellen, Stromazellen, Makrophagen, T-Zellen und B-Zellen.

7. Verfahren nach einem der Ansprüche 1 bis 6, wobei die Farbstoffdomäne A das Gewebe oder der Bereich eines digitalen Bilds eines Gewebes ist, der sich für einen zelltypspezifischen Marker anfärbt.

8. Verfahren nach einem der Ansprüche 1 bis 7, wobei das neuronale Faltungsnetzwerk als ein Diskriminator in dem Zyklus fungiert, der durch zwei generativ-adversativen Netzwerke ausgebildet wird.

9. System, das eine Bewertung für eine histopathologische Diagnose für einen Krebspatienten generiert, umfassend: Code zum Laden eines ersten Bildausschnitts in eine Verarbeitungseinheit, wobei der erste Bildausschnitt aus einem digitalen Bild einer Gewebescheibe geschnitten wird und wobei die Gewebescheibe immunhistochemisch unter Verwendung eines diagnostischen Antikörpers angefärbt wurde; Code zum Bestimmen, wie viele Pixel des ersten Bildausschnitts zu einem ersten Gewebe gehören, das durch den diagnostischen Antikörper positiv angefärbt wurde, wie viele Pixel des ersten Bildausschnitts zu einem zweiten Gewebe gehören, das durch den diagnostischen Antikörper negativ angefärbt wurde, und wie viele Pixel des ersten Bildausschnitts zu einem dritten Gewebe gehören, das weder das erste Gewebe noch das zweite Gewebe ist, wobei das Bestimmen durch Verarbeiten des ersten Bildausschnitts unter Verwendung eines neuronalen Faltungsnetzwerks erfolgt; Code zum Verarbeiten, unter Verwendung des neuronalen Faltungsnetzwerks, zusätzlicher Bildausschnitte, die aus dem digitalen Bild geschnitten wurden, um zu bestimmen, wie viele Pixel jedes zusätzlichen Bildausschnitts zu dem ersten Gewebe gehören, wie viele Pixel jedes zusätzlichen Bildausschnitts zu dem zweiten Gewebe gehören und wie viele Pixel jedes zusätzlichen Bildausschnitts zu dem dritten Gewebe gehören; Code zum Berechnen der Auswertung der histopathologischen Diagnose basierend auf einer Gesamtzahl von Pixeln, die zu dem ersten Gewebe gehören; und eine grafische Benutzeroberfläche, die das digitale Bild und die Auswertung anzeigt, wobei das neuronale Faltungsnetzwerk unter Verwendung von generativ-adversativem Lernen trainiert wird, wobei das neuronale Faltungsnetzwerk unter Verwendung zweier generativ-adversativer Netzwerke trainiert wird, die einen Zyklus ausbilden, wobei ein erstes der zwei generativ-adversativen Netzwerke Bildausschnitte, die auf einer Farbstoffdomäne A generiert werden, in gefälschte Ausschnitte eine Farbstoffdomäne B transformiert und wobei ein zweites der zwei generativ-adversativen Netzwerke Bildausschnitte, die auf der Farbstoffdomäne B generiert werden, in gefälschte Ausschnitte der Farbstoffdomäne A transformiert, wobei das erste der zwei generativ-adversativen Netzwerke die Segmentierung auf der Farbstoffdomäne B nach der Transformation der Bildausschnitte, die auf der Farbstoffdomäne A generiert werden, in gefälschte Ausschnitte der Farbstoffdomäne B durchführt, und wobei das zweite der zwei generativ-adversativen Netzwerke die Segmentierung auf der Farbstoffdomäne A, nach der Transformierung der Bildausschnitte, die auf der Farbstoffdomäne B generiert werden, in gefälschte Ausschnitte der Farbstoffdomäne A durchführt, wobei die Auswertung ein Verhältnis der Gesamtzahl von Pixeln, die zu dem ersten Gewebe gehören, zu einer Gesamtzahl von Pixeln ist, die entweder zu dem ersten Gewebe oder zu dem zweiten Gewebe gehören, wobei die Farbstoffdomäne A das Gewebe oder der Bereich eines digitalen Bilds eines Gewebes ist, der für einen spezifischen Biomarker oder Farbstoff angefärbt ist, und wobei die Farbstoffdomäne B das Gewebe oder der Bereich des digitalen Bilds des Gewebes ist, der für den diagnostischen Antikörper angefärbt ist, und wobei sowohl das erste als auch das zweite Gewebe für den spezifischen Biomarker oder Farbstoff gefärbt sind.

10. System nach Anspruch 9, wobei, falls die Auswertung größer als ein zuvor bestimmter Schwellenwert ist, dann das System eine Therapie empfiehlt, bei der ein therapeutischer Antikörper verwendet wird, der auf dasselbe Protein wie das, auf das durch den diagnostischen Antikörper abgezielt wird, abzielt.

11. System nach Anspruch 10, wobei der diagnostische Antikörper ein PD-L1 ist und wobei bei der Therapie ein Arzneimittel verwendet wird, das auf ein Protein der Gruppe abzielt, bestehend aus PD-L1 und PD-1.

**Revendications**

1. Procédé de génération d'un score d'un diagnostic histopathologique d'un patient atteint de cancer, comprenant :

le chargement d'un premier patch d'image dans une unité de traitement, dans lequel le premier patch d'image est découpé à partir d'une image numérique d'une tranche de tissu, et dans lequel la tranche de tissu a été colorée par immunohistochimie à l'aide d'un anticorps diagnostique ;
la détermination de combien de pixels du premier patch d'image appartiennent à un premier tissu qui a été coloré positivement par l'anticorps diagnostique, de combien de pixels du premier patch d'image appartiennent à un deuxième tissu qui a été coloré négativement par l'anticorps diagnostique, et de combien de pixels du premier patch d'image appartiennent à un troisième tissu qui n'est ni le premier tissu ni le deuxième tissu, dans lequel la détermination est effectuée par traitement du premier patch d'image à l'aide d'un réseau neuronal convolutif ;
le traitement, à l'aide dudit réseau neuronal convolutif, de patchs d'image supplémentaires qui ont été découpés à partir de l'image numérique pour déterminer combien de pixels de chaque patch d'image supplémentaire appartiennent au premier tissu, combien de pixels de chaque patch d'image supplémentaire appartiennent au deuxième tissu, et combien de pixels de chaque patch d'image supplémentaire appartiennent au troisième tissu ;

le calcul du score du diagnostic histopathologique en fonction d'un nombre total de pixels qui appartiennent au premier tissu ; et

l'affichage de l'image numérique et du score sur une interface utilisateur graphique,

dans lequel le réseau neuronal convolutif est entraîné à l'aide d'un apprentissage antagoniste génératif,

dans lequel le réseau neuronal convolutif est entraîné à l'aide de deux réseaux antagonistes génératifs formant un cycle, dans lequel un premier des deux réseaux antagonistes génératifs transforme des patchs d'image générés sur un domaine de coloration A en faux patchs d'un domaine de coloration B, et dans lequel un second des deux réseaux antagonistes génératifs transforme des patchs d'image générés sur le domaine de coloration B en faux patchs du domaine de coloration A,

dans lequel le premier des deux réseaux antagonistes génératifs effectue une segmentation sur le domaine de coloration B après la transformation des patchs d'image générés sur le domaine de coloration A en faux patchs du domaine de coloration B, et dans lequel le second des deux réseaux antagonistes génératifs effectue une segmentation sur le domaine de coloration A après la transformation des patchs d'image générés sur le domaine de coloration B en faux patchs du domaine de coloration A

dans lequel le score est un rapport entre le nombre total de pixels qui appartiennent au premier tissu et un nombre total de pixels qui appartiennent au premier tissu ou au deuxième tissu, dans lequel le domaine de coloration A est le tissu ou la région d'une image numérique d'un tissu qui se colore pour un biomarqueur ou une coloration spécifique et dans lequel le domaine de coloration B est le tissu ou la région de l'image numérique du tissu qui se colore pour l'anticorps diagnostique, et

dans lequel le premier et le deuxième tissu se colorent tous les deux pour le biomarqueur ou la coloration spécifique.

2. Procédé selon la revendication 1, dans lequel la tranche de tissu est prise à partir d'une biopsie de cancer du poumon.

3. Procédé selon la revendication 1 ou la revendication 2, dans lequel l'anticorps diagnostique est pris dans le groupe constitué de : HER2, PD-L1, PD-L2, et CD73.

4. Procédé selon l'une quelconque des revendications 1 à 3, dans lequel l'anticorps diagnostique cible une protéine, comprenant en outre :
le choix d'une thérapie si le score obtenu à l'aide de l'anticorps diagnostique est supérieur à un seuil prédéterminé, dans lequel la thérapie qui est choisie utilise un anticorps thérapeutique qui se lie à la protéine ciblée par l'anticorps diagnostique.

5. Procédé selon la revendication 4, dans lequel l'anticorps diagnostique est PD-L1, et dans lequel la thérapie choisie utilise un traitement PD-L1 si le score est supérieur au seuil prédéterminé.

6. Procédé selon l'une quelconque des revendications 1 à 5, dans lequel le premier tissu qui a été coloré positivement par l'anticorps diagnostique comporte des cellules prises dans le groupe constitué de : cellules épithéliales, cellules endothéliales, cellules stromales, macrophages, cellules T et cellules B.

7. Procédé selon l'une quelconque des revendications 1 à 6, dans lequel le domaine de coloration A est le tissu ou la région d'une image numérique d'un tissu qui se colore pour un marqueur spécifique de type cellulaire.

8. Procédé selon l'une quelconque des revendications 1 à 7, dans lequel le réseau convolutif agit en guise de discriminateur dans le cycle formé par les deux réseaux antagonistes génératifs.

9. Système qui génère un score d'un diagnostic histopathologique pour un patient atteint de cancer, comprenant : un code pour le chargement d'un premier patch d'image dans une unité de traitement, dans lequel le premier patch d'image est découpé à partir d'une image numérique d'une tranche de tissu, et dans lequel la tranche de tissu a été colorée par immunohistochimie à l'aide d'un anticorps diagnostique ; un code pour la détermination de combien de pixels du premier patch d'image appartiennent à un premier tissu qui a été coloré positivement par l'anticorps diagnostique, de combien de pixels du premier patch d'image appartiennent à un deuxième tissu qui a été coloré négativement par l'anticorps diagnostique, et de combien de pixels du premier patch d'image appartiennent à un troisième tissu qui n'est ni le premier tissu ni le deuxième tissu, dans lequel la détermination est effectuée par traitement du premier patch d'image à l'aide d'un réseau neuronal convolutif ; un code pour le traitement, à l'aide dudit réseau neuronal convolutif, de patchs d'image supplémentaires qui ont été découpés à partir de l'image numérique pour déterminer combien de pixels de chaque patch d'image supplémentaire appartiennent au premier tissu, combien de pixels de chaque patch d'image supplémentaire appartiennent au deuxième tissu, et combien de pixels de chaque patch d'image supplémentaire appartiennent au troisième tissu ; un code pour le calcul du score du diagnostic histopathologique en fonction d'un nombre total de pixels qui appartiennent au premier tissu ; et une interface utilisateur graphique qui affiche l'image numérique et le score, dans lequel le réseau neuronal convolutif est entraîné à l'aide d'un apprentissage antagoniste génératif, dans lequel le réseau neuronal convolutif est entraîné à l'aide de deux réseaux antagonistes génératifs formant un cycle, dans lequel un premier des deux réseaux antagonistes génératifs transforme des patchs d'image générés sur un domaine de coloration A en faux patchs d'un domaine de coloration B, et dans lequel un second des deux réseaux antagonistes génératifs transforme des patchs d'image générés sur le domaine de coloration B en faux patchs du domaine de coloration A, dans lequel le premier des deux réseaux antagonistes génératifs effectue une segmentation sur le domaine de coloration B après la transformation des patchs d'image générés sur le domaine de coloration A en faux patchs du domaine de coloration B, et dans lequel le second des deux réseaux antagonistes génératifs effectue une segmentation sur le domaine de coloration A après la transformation des patchs d'image générés sur le domaine de coloration B en faux patchs du domaine de coloration A, dans lequel le score est un rapport entre le nombre total de pixels qui appartiennent au premier tissu et un nombre total de pixels qui appartiennent au premier tissu ou au deuxième tissu, dans lequel le domaine de coloration A est le tissu ou la région d'une image numérique d'un tissu qui se colore pour un biomarqueur ou une coloration spécifique et dans lequel le domaine de coloration B est le tissu ou la région de l'image numérique du tissu qui se colore pour l'anticorps diagnostique, et dans lequel le premier et le deuxième tissu se colorent tous les deux pour le biomarqueur ou la coloration spécifique.

10. Système selon la revendication 9, dans lequel si le score est supérieur à un seuil prédéterminé, alors le système recommande une thérapie qui utilise un anticorps thérapeutique qui cible la même protéine que celle ciblée par l'anticorps diagnostique.

11. Système selon la revendication 10, dans lequel l'anticorps diagnostique est PD-L1, et dans lequel la thérapie utilise un médicament ciblant une protéine du groupe constitué de PD-L1, PD-1.

FIG. 1

FIG. 2

NON-
EPITHELIUM
REGION OF
TISSUE
—22

TUMOR
EPITHELIUM
STAINED WITH
CYTOKERATIN
21

NON-
EPITHELIUM
REGION OF
TISSUE
22

FIG. 3

NON-
EPITHELIUM
REGION OF
TISSUE
—22

NON-
EPITHELIUM
REGION OF
TISSUE
22

TUMOR
EPITHELIUM
STAINED WITH
CYTOKERATIN
21

—21
TUMOR
EPITHELIUM
STAINED WITH
CYTOKERATIN

FIG. 4

FIG. 5

FIG. 6

FIG. 8

FIG. 9A

FIG. 9B

FIG. 9C

FIG. 10A
FIG. 10B
FIG. 10C

Fig. 10 $\mathcal{D}$

(a) Two class segmentation

(b) Three class segmentation

Fig. 11

Fig. 12

(a)          (b)

Fig.13

Fig. 14

Fig. 15

Fig. 16

Fig. 17

(a) Lin's Correlation Coefficient

(b) Pearson Correlation Coefficient

(c) Mean Absolute Error

Fig. 18

(a) Lin's Correlation Coefficient

(b) Pearson Correlation Coefficient

(c) Mean Absolute Error

Fig. 19

(a) Overall Percent Agreement

(b) Negative Percent Agreement

(c) Positive Percent Agreement

Fig. 20

Fig. 21

| ObjectClass | count | count_1 | count_2 |
|---|---|---|---|
| -1 | 20,613 | (undefined) | (undefined) |
| 1 | 24,154 | 29,027 | 28,519 |
| 2 | 24,221 | 24,998 | 32,278 |
| 3 | 1,861 | 9,998 | 1,955 |
| 4 | 979 | 4,590 | 2,091 |
| 5 | 1,619 | 2,822 | 1,712 |
| 6 | 31,374 | 33,027 | 33,485 |
| 7 | 7,076 | 7,455 | 11,877 |
| 8 | 63,025 | 63,025 | 63,025 |

Fig. 22

Fig. 23

Fig. 24

*image*   $I$   $x$   $\hat{y}$   *class*

Fig. 25

*image*   $I$   $x$   $\hat{I}$   *reconstructed image*

Fig. 26

*image*   $I$   $x$   $\hat{I}$   *reconstructed image*

$\hat{y}$   *class*

Fig. 27

Fig. 28

Fig. 29

Fig. 30

Fig. 31

Fig. 32

loss/g_loss

Generator loss

loss/d_loss

Discriminator loss

loss/c_loss

Classifier loss

Fig. 33

TC(-), TC(+), Other

Fig. 34

TC(-), TC(+), Other

Fig. 35

Fig. 36

Fig. 37

Fig. 38

Fig. 39

Fig. 40

Fig. 41

Fig. 42

Fig. 43

**EP 3 588 382 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **HERNÁNDEZ, C. X.** ; **SULTAN, M. M.** ; **PANDE, V. S**. Using deep learning for segmentation and counting within microscopy data. *arXiv preprint arXiv: 1802.10548*, 2018

- **LONG, J.** ; **SHELHAMER, E.** ; **DARREL, T**. Fully convolutional networks for semantic segmentation. *CoRR, abs/1411.4038*, 2014
- **NOH, H.** ; **HONG, S.** ; **HAN, B.** Learning deconvolution network for semantic segmentation. *ICCV*, 2015, 1520-1528